# EUROPEAN PATENT APPLICATION

(11) **EP 3 839 072 A1**
(43) Date of publication of application: **23.06.2021**
(21) Application number: 19306664.4
(22) Date of filing: 17.12.2019
(51) Int. Cl.: C12Q 1/689, A61K 35/74

(54) **BACTERIAL COMBINATIONS PREDICTIVE OF THE ACTIVITY OF MULTIPLE SCLEROSIS**

(71) Applicant: Luxia Scientific, 77590 Bois le Roi (FR)
(72) Inventor: CERVINO, Alessandra, 77590 BOIS LE ROI (FR)
(74) Representative: Regimbeau

(57) **Abstract**

Multiple Sclerosis or MS is an autoimmune disease that affects the brain, spinal cord, and the optic nerves of the central nervous system (CNS). Surrounding and protecting the nerve fibers of the CNS is a fatty tissue called myelin which helps nerve fibers conduct electrical impulses. In MS, myelin is lost in multiple areas, leaving scar tissue called sclerosis. These damaged areas are also known as plaques or lesions. Sometimes the nerve fiber itself is damaged or broken. When myelin or the nerve fiber is destroyed or damaged, the ability of the nerves to conduct electrical impulses to and from the brain is disrupted, and this produces the various symptoms of MS.

## Description

Multiple Sclerosis or MS is an autoimmune disease that affects the brain, spinal cord, and the optic nerves of the central nervous system (CNS). Surrounding and protecting the nerve fibers of the CNS is a fatty tissue called myelin which helps nerve fibers conduct electrical impulses. In MS, myelin is lost in multiple areas, leaving scar tissue called sclerosis. These damaged areas are also known as plaques or lesions. Sometimes the nerve fiber itself is damaged or broken. When myelin or the nerve fiber is destroyed or damaged, the ability of the nerves to conduct electrical impulses to and from the brain is disrupted, and this produces the various symptoms of MS.

People with MS can expect one of four clinical courses of disease, each of which might be mild, moderate, or severe. These include Relapsing-Remitting (RR), Primary-Progressive (PP), Secondary-Progressive (SP), and Progressive-Relapsing (PR). Individuals with RR-MS experience clearly defined flare-ups (also called relapses, attacks, or exacerbations). These are episodes of acute worsening of neurologic function. They are followed by partial or complete recovery periods (remissions) free of disease progression. Individuals with PP-MS experience a slow but nearly continuous worsening of their disease from the onset, with no distinct relapses or remissions. However, there are variations in rates of progression over time, occasional plateaus, and temporary minor improvements. Individuals with SP-MS experience an initial period of relapsing-remitting disease, followed by a steadily worsening disease course with or without occasional flare-ups, minor recoveries (remissions), or plateaus. Individuals with PR-MS experience a steadily worsening disease from the onset but also have clear acute relapses (attacks or exacerbations), with or without recovery. In contrast to RR-MS, the periods between relapses are characterized by continuing disease progression. Symptoms can include muscle weakness, visual disturbances, balance problems, memory loss, and/or loss of bowel or bladder control.

Patients can progress rapidly over several months to death, or may have a few relapses and then remain clinically stable for many decades. It is difficult to predict which patients will progress and which will remain relatively stable. Although there are clearly patients in whom the disease remains benign, it is very difficult to predict which course a patient's disease will follow.

The McDonald criteria was introduced in 2001, and revised in 2005 (Polman et al., 2006, Ann Neurol. ;59(4):727-8), as guidelines to facilitate early and accurate diagnosis of multiple sclerosis (MS). Diagnostic classifications are reduced to a) having MS, b) not having MS, or c) having possible MS. Advantages to the criteria include the capability of making a definitive diagnosis of MS either after a monosymptomatic presentation or in the context of a primary progressive course. However, the diagnostic classification scheme and MRI criteria remain complicated and tedious, and this complexity limits their use in everyday practice. Furthermore, the specificity of these criteria is relatively low, emphasizing the importance of clinical judgment in excluding other diagnoses. In addition, studies have observed that standard MS disease-modifying medications can benefit patients who do not yet fulfil these diagnostic criteria.

Today, drugs used for multiple sclerosis drug therapy can be divided into two groups: drugs for symptomatic treatments, and drugs modifying the course of multiple sclerosis. Drugs used for symptomatic treatment of multiple sclerosis include steroids, such as, glucocorticoids. While the interferons and Copaxone® are clearly beneficial, the effect of treatment diminishes over time, and a meaningful number of patients do not respond to one or more of the currently available options.

Currently there are no reliable clinical, genetic, biological and/or immunological markers that accurately diagnose MS, clinically characterize MS, and forecast a response to MS therapy. Therefore, the clinicians often approach treatment on a trial and error basis, heavily weighing patient tolerance for side effects and choice of administration versus efficacy. The reliable diagnosis and characterization of MS, and a prognostic indicator of the clinical response to one or more therapies for the treatment of MS, would be very valuable.

Also, there are no known methods for predicting or diagnosing if a patient suffering from multiple sclerosis is about to experience a relapse. Such a prediction or diagnosis would help the patient and the doctor to monitor the progress of the relapse, and prevent the relapse from being a severe relapse.

The incomplete understanding of drug mechanisms of action together with disease heterogeneity means that there are no methods of identifying patient suitability for the various biologics prior to the initiation of the treatment. Establishing a rational basis on which to select patients for specific biologics would help patients to be treated more efficiently; those that would be likely to respond would initiate the biologic in question whereas those unlikely to respond could be provided with another treatment.

In absence of reliable literature on efficacy and safety of biologics and given the percentage of patients that do not respond or experience severe adverse effects, the destructive nature of MS, and the societal costs of inefficacious biological treatments, there is a strong need to make predictions on success before starting the therapy. Ideally, a molecular biomarker signature as a predictor for therapy responsiveness should be obtained prior to the start of therapy in a readily available bio-sample, such as stool.

Ultimately, this may lead to a personalized form of medicine, whereby the best suited therapy will be applied to an individual patient.

The present inventors herein propose a diagnostic test based on the analysis of the bacterial content of stool samples obtained from MS patients. This process provides a high safety, because the flora samples can be obtained in a non-invasive manner, and then transported at room temperature, so that the convenience of detecting is greatly improved.

In the past, some studies have reported that patients with RR-MS have a gut microbiota that has, as compared with healthy controls, higher amounts of bacteria of the genus *Pedobacteria*, *Flavobacterium*, *Pseudomonas*, *Mycoplana*, *Acinetobacter*, *Eggerthella*, *Dorea*, *Blautia*, *Streptococcus* and *Akkermansia.* By contrast, MS patients have been shown to have a gut microbiota with impoverished microbial populations of *Prevotella*, *Bacteroides*, *Parabacteroides*, *Haemophilus*, *Sutterella*, *Adlercreutzia*, *Coprobacillus*, *Lactobacillus*, *Clostridium*, *Anaerostipes* and *Faecalibacterium* (Schepici G. et al, 2019).

Yet, the results obtained from these prior studies are somewhat contradictory, and there is presently no consensus on the number of bacteria genus to be detected, their nature nor their association to a particular stage of the MS disease.

A number of factors might influence the sensitivity and reproducibility range from sample differences, variation in amount and quality of starting RNA material, amplification and labeling strategies and dyes, to probe sequence and hybridization conditions. In addition, the lack of standardized approaches for normalization and usage of data analysis algorithms can also influence the outcome. Furthermore, most microarray studies are not prospectively planned and often do not have detailed protocols, but rather tend to make use of existing samples. Therefore, verification of results is an essential step in microarrays studies and quality criteria have to be set.

### DETAILED DESCRIPTION OF THE INVENTION

Meta-analyses of multiple gene expression microarray datasets provide discriminative gene expression signatures that are identified and validated on a large number of microarray samples, generated by different laboratories and microarray technologies. Predictive models generated by this approach are better validated than those generated on a single data set, while showing high predictive power and improved generalization performance.

In the present invention, the meta-analysis was performed according to the stepwise approach in conducting meta-analysis on microarray datasets (1-identify suitable microarray studies; 2-extract data from studies (this step also involved getting additional information from the authors of selected studies); 3-prepare the individual datasets; 4-annotate the individual datasets; 5-resolve the many-to-many relationship between probes and genes; 6-combine the study-specific estimates; 7-analyze, present, and interpret results) described in Ramasamy *et al.*

Six bacteria differentially present in the stool of the MS patients (versus in healthy patients) have thereby been identified. These bacteria are of the genus *Parabacteroides, Collinsella*, *Subdoligranulum*, *Anaerostipes*, *Akkermansia* and *Lactococcus.*

The present inventors propose to detect the presence and abundance of these six bacteria in the stool of the patient, in order to establish the activity of the MS disease in a rapid, non-invasive and efficient manner.

### Definitions

Unless otherwise stated, the following terms used in this application, including the specification and claims, have definitions given below.

As used herein, the term "multiple sclerosis" or "MS" refers to an autoimmune disease affecting the central nervous system. MS is a disease of unknown aetiology with a prolonged course involving many remissions and relapses. In some embodiments, individuals with multiple sclerosis experience a wide range of symptoms, including, but not limited to, double vision, blindness in one eye, muscle weakness, trouble with sensation, or trouble with coordination. Several forms of multiple sclerosis are known, and the terms "multiple sclerosis" or "MS", as used herein, is meant to include all such forms. Some commonly known forms of MS are benign multiple sclerosis (benign MS), relapsing-remitting multiple sclerosis (RRMS), secondary progressive multiple sclerosis (SPMS), primary progressive multiple sclerosis (PPMS), and progressive-relapsing multiple sclerosis (PRMS). In the relapsing forms of MS, such as, but not limited to, relapsing-remitting multiple sclerosis and progressive-relapsing multiple sclerosis symptoms may occur in isolated attacks known as relapses, attacks, crisis, exacerbation, or flare.

The terms "relapse", "attack", "crisis", "exacerbation" or "flare" as used herein refers to an increase in the severity of a disease or any of its signs or symptoms. In some embodiments the relapses last at least 24 hours. In some embodiments, the relapses may be associated with inflammation or demyelination in the brain or spinal cord. In some embodiments, the exacerbations last from a few days to several weeks, or several months. In some embodiments, the relapses are separated from the previous relapse by a period ranging from few days, or few weeks, or few months.

The term "treatment period" refers to the length of the time period wherein an individual is undergoing treatment for a disease. Similarly, the term "monitoring period" refers to the length of time wherein the progress of a disease or recovery from a disease in an individual is being monitored. During the treatment period or monitoring period, the individual may be under constant supervision of medical personnel or intermittent supervision.

As used herein, the terms "treatment", "treating", and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse affects attributable to the disease. "Treatment", as used herein, covers any treatment of a disease in a mammal, particularly in a human, and includes: (a) preventing the disease from occurring in a subject which may be predisposed to the disease but has not yet been diagnosed as having it; (b) inhibiting the disease, i.e., arresting its development; and (c) relieving the disease, i.e., causing regression of the disease.

In accordance with various embodiments herein, the term "control value" is used. This value could be particular to the specific individual under examination or treatment of a disease, such that it could be established, for example, by a physician examining stool values of a patient when a patient appears to be in remission or have few symptoms of disease, and thus create a baseline or value of a baseline for that patient or individual that can later serve as a future control value for that same patient or individual. Alternatively, and preferably, the control value can be obtained from a healthy subject or from a group of healthy subjects.

As used herein, the term "gene" refers broadly to a genetic information unit. It is composed of DNA or RNA that may code for a polypeptide or for an RNA chain of a given organism. More specifically, a gene is a locatable region of genomic sequence, which is associated with regulatory regions, transcribed regions, and /or other functional sequence regions. The genes which are referred to in this invention are preferably "bacterial genes", i.e., they correspond to a locatable region of the genome of a bacterium. More preferably, these genes are ribosomal genes of the genome of bacteria, for example the genes encoding the ribosomal 16S RNA. These genes have indeed a genus-dependent sequence that is well-known and can easily be tested by conventional means.

16S rRNA gene sequences contain hypervariable regions (V1-V9) ranging from about 30 to 100 base pairs long, that can provide genus-specific signature sequences useful for identification of bacteria (Pereira F. et al, 2010). Analysis of the hypervariable regions of this gene, notably the V3-V4 regions of this gene, remains one of the most useful tools available to bacterial community studies (Jovel J. et al, 2016).

In all the present description, the term "bacterial genus signature" refers to a set of information that is related to the amount of one or more bacterial genus present in a tested sample. This information may arise from the identification of the amount of gene copies or gene products or gene fragments specific of bacteria belonging to a particular bacteria genus, in the tested sample. As used herein, the "bacterial genus signature of the invention" (also called the "signature of the invention") therefore encompasses the set of bacterial genera identified by the inventors, said set being revealed by either the expression level or by the abundance of one or more gene(s) or gene fragment(s) that is (are) present specifically in bacteria of each genus.

"Gene fragments" within the scope of the invention are locatable part of the bacterial genomes, whose size do not exceed 1000 base pair. These fragments are preferably hypervariable regions that are commonly used to distinguish different bacterial genera. For example, these fragments can be the hypervariable regions V1-V9, in particular the V3-V4 regions, of the gene encoding the ribosomal 16S RNA.

As used herein, the "expression" of a gene is the process by which information from a gene is used in the synthesis of a functional gene product. These products are often proteins, but in non-protein coding genes such as ribosomal RNA (rRNA), transfer RNA (tRNA) or small nuclear RNA (snRNA) genes, the product is also a functional RNA. Gene expression can thus be detected by determining the presence of the corresponding rRNA, tRNA, mRNA, snRNA and / or the gene products at the protein level, by conventional means.

On another hand, "gene / fragment abundance" refers to the absolute or relative amount of the tested genes or fragments. "Absolute amount" (or "absolute abundance") of a gene / fragment designates the total number of copies of said gene / fragment in a define volume of the tested sample, whereas "relative amount" (or "relative abundance") of a gene / fragment designates the total number of copies of said gene / fragment relative to the total amount of genes or alternatively the total number of copies of said gene / fragment relative to the amount of a single reference gene / fragment or preferably a combination of reference genes / fragments present in the tested sample.

In a preferred embodiment, the bacterial genus signature of the invention is revealed by detecting the absolute or relative abundance of a bacterial genus within the tested sample. "Absolute amount" (or "absolute abundance") of a bacterial genus designates the total number of copies of at least one gene (or gene fragment) that is specifically expressed in the bacteria of said bacterial genus, in a define volume of the tested sample. Conversely, the "relative amount" (or "relative abundance") of a bacterial genus designates the number of copies of at least one gene (or gene fragment) that is specifically present in the bacteria of said bacterial genus, relative to the total amount of bacterial genes (or gene fragments) present in the sample (in practice, to the sum of all the bacterial genes or gene fragments present in the sample). It can also designate the number of copies of at least one gene (or gene fragment) that is specifically present in the bacteria of said bacterial genus, relative to the amount of a reference bacterial gene (or fragment) present in the tested sample. Said reference bacterial gene (or fragment) can be for example a bacterial gene (or fragment) that is conserved and identical in all the bacterial genus present in the gut or in a stool sample, or a combination of such conserved bacterial genes (or fragments).

In a more preferred embodiment, the relative abundance of each bacterial genus belonging to the signature of the invention is detected by measuring the abundance of the hypervariable region of the gene encoding the bacterial ribosomal 16S rRNA for said bacterial genus, relative to the total abundance of fragments from the hypervariable region V3-V4 of the gene encoding the ribosomal 16S rRNA (whatever the genus).

It is possible to use another conserved region or ubiquitous gene to normalise the abundance of the tested genus. Ubiquitous genes are for example DNA polymerase genes, or genes coding for proteins involved in glucose metabolism, that are often used as reference genes in metagenomic studies.

By "abundance of a gene fragment or gene", it is meant the absolute or the relative abundance of a gene fragment or gene. This abundance can be determined by detecting the copies number of the tested gene fragments or genes at the DNA level, for example by quantitative PCR, sequencing or nucleic acid microarrays.

By "expression of a gene", it is meant the expression levels of said gene. These expression levels can be determined by detecting the levels of the gene products, for example the transcript levels or the protein levels corresponding to the tested genes.

In the context of the invention, two genes (or gene fragments) are held "equivalent" if the replacement of one gene (or gene fragment) by the other does not significantly affect the performance of the method of the invention. Such equivalent genes (or gene fragments) are in fact concomitantly absent from the samples and their abundance vary concomitantly, in the same direction and in the same proportion in the samples where they are present. This is typically the case when "gene A" is correlated to "gene B", meaning that the expression level or abundance of "gene A" is statistically correlated to the expression level or abundance of "gene B" respectively. In the context of the invention, this correlation is rather positive (meaning that when "gene A" is upregulated in a patient, then "gene B" is also upregulated in that same patient). This correlation can be determined for example by a measure of association such as the Pearson's or Spearman's correlation coefficient. Alternatively, covariance can be used for the identification of equivalent genes.

In the context of the present invention, "covariant" genes (or gene fragments) are typically linked with a minimum Pearson correlation of at least 0.8, more preferably of 0.9, to the representative genes (or gene fragments) disclosed in the present invention.

The equivalence of two genes (or gene fragments) is expected by the man skilled in the art to be the consequence of situations such as when two genes (or gene fragments) belong to the same genome (Qin J, et al., Nature, 2012). In the context of the invention, said equivalent gene (or fragment) is a covariant gene (or fragment) belonging to the same bacterium.

Therefore, a bacterial genus contains representative genes or fragments (see below) and hundreds of equivalent genes or fragments, that can be identified by testing their covariance with the representative genes.

For simplification' purposes, the present application only discloses the sequences of few gene fragments for each bacteria genus. These gene fragments are hereafter referred to as "representative gene fragments". Their sequences are referred to as "representative sequences".

These representative gene fragments correspond to parts of the hypervariable regions of the gene coding for the ribosomal 16S RNA. These representative sequences are unique for each bacteria genus (i.e., these particular sequences can be detected only in bacteria of said genus). They enable to efficiently detect the presence of said genus in a sample with great sensitivity.

The sequences of these representative gene fragments are displayed in :
- SEQ ID NO: 1 to 8 corresponding respectively to the eight fragments: AB261128|S000727759, AY974070|S000577391, AB238922|S000650586, AB574482|S003620294, AB470343|S001291847, AB238928|S000650592, AB739697|S003286972, and JN029805|S002915318 of the ribosomal 16S RNA encoding gene that are specifically present in the bacteria of the genus *Parabacteroides.*
- SEQ ID NO: 9-12 corresponding respectively to the four fragments: AB031063|S000382905, AB490807|S001587406, AB011816|S000382127, and AB031061|S000382903 of the ribosomal 16S RNA encoding gene that are specifically present in the bacteria of the genus *Collinsella.*
- SEQ ID NO: 13 corresponding to the fragment AJ518869|S000416814 of the ribosomal 16S RNA encoding gene that is specifically present in the bacteria of the genus *Subdoligranulum.*
- SEQ ID NO: 14-19 corresponding respectively to the six fragments FR749934|S002350797, FJ947528|S001549427, AJ270487|S000088221, FR749932|S002350795, JF412658|S002447232 and JX273468|S003613922 of the ribosomal 16S RNA encoding gene that are specifically present in the bacteria of the genus *Anaerostipes.*
- SEQ ID NO: 20, corresponding to the fragment AY271254|S000460661 of the ribosomal 16S RNA encoding gene that is specifically present in the bacteria of the genus *Akkermansia.*
- SEQ ID NO: 21-33, corresponding respectively to the thirteen fragments AB100803|S000439499, AB100804|S000439500, AB681296|S003263187, EF694029|S000893640, DQ343754|S000640753, EU770697|S001153507, AB100802|S000439498, AB485959|S002949140, AB598994|S003316271, AB699722|S003265167, AB775178|S003659973, EF694030|S000893641, and EF694028|S000893639 of the ribosomal 16S RNA encoding gene that are specifically present in the bacteria of the genus *Lactococcus.*

The present invention can be implemented by using any covariant fragments of these representative fragments, for each bacterial genus (these covariant fragments are in fact "equivalent" to these representative genes). Thus, the methods and kits of the invention can use any covariant fragment of the representative fragments SEQ ID NO:1-33.

More generally, the methods and kits of the invention can use any representative gene that is specifically expressed in one of the tested bacterial genus, or any covariant gene thereof.

By "specifically", it is herein meant that the target gene or fragment is present only in the genome of the target bacterial genus. In other words, the sequence of said target gene or fragment cannot be detected in the genome of bacteria belonging to other genera.

As used herein, a sequence (of a gene or gene fragment) is "specific" for a bacterial genus if it enables one to certainly conclude that the sample contains a certain amount of bacteria of said bacterial genus. This means that this sequence is found only in the genome of bacteria of said bacterial genus. This also means that it is possible to identify sequences having at least 98% identity with this specific sequence only in bacteria of said bacterial genus.

The "bacterial genus signature" of the invention comprises or consists of:
(i) The bacteria genus *Parabacteroides,* and
(ii) The bacteria genus *Collinsella*, and
(iii) The bacteria genus *Subdoligranulum*, and
(iv) The bacteria genus *Anaerostipes*, and
(v) The bacteria genus *Akkermansia*,
   and
(vi) The bacteria genus *Lactococcus,*
since these bacterial genera have been demonstrated to be particularly relevant for assessing the risk of MS relapse of a subject.

The "bacterial genus signature" of the invention contains, in addition to or in replacement of representative sequences, equivalent genes or fragments which confer the same performance to the method of the invention.

In particular, the signature of the invention comprises or consists of:
(i) A representative sequence of the bacterial genus *Parabacteroides,* or a covariant sequence thereof, and
(ii) A representative sequence of the bacterial genus *Collinsella,* or a covariant sequence thereof, and
(iii) A representative sequence of the bacterial genus *Subdoligranulum,* or a covariant sequence thereof, and
(iv) A representative sequence of the bacterial genus *Anaerostipes*, or a covariant sequence thereof, and
(v) A representative sequence of the bacterial genus *Akkermansia* or a covariant sequence thereof, and
(vi) A representative sequence of the bacterial genus *Lactococcus,* or a covariant sequence thereof,
since these bacterial genera have been demonstrated to be particularly relevant for assessing the risk of MS relapse of a subject.

The representative sequences listed in i) to vi) are for example SEQ ID NO: 1-8, SEQ ID NO: 9-12, SEQ ID NO: 13, SEQ ID NO: 14-19, SEQ ID NO: 20, and SEQ ID NO: 21-33.

The "signature of the invention" may contain any of these representative sequences (SEQ ID NO: 1-33), or any covariant sequences thereof, or combinations of representative and covariant sequences. Alternatively, it may contain the arithmetic or geometric mean of the signals associated with several or all the equivalent sequences of each genus.

By "comprising", it is intended to mean that the signature of the invention may further comprise any other genes or fragments, among which, specific genes or fragments that do not significantly affect the essential characteristics of the signature of the invention (therefore overlapping the meaning of the term "consisting essentially of"). In contrast, by "consisting of", it is intended to mean that no further gene or fragment is present in the signature which is analysed.

As used herein, the term "pharmaceutically acceptable carrier" includes any and all solvents, dispersion media, coatings, antibacterial and antifungal agents, isotonic and absorption delaying agents, and the like that are physiologically compatible. Examples of pharmaceutically acceptable carriers or pharmaceutical carrier include one or more of water, saline, phosphate buffered saline, dextrose, glycerol, ethanol and the like, as well as combinations thereof. In many cases, it can be preferable to include isotonic agents, for example, sugars, polyalcohols such as mannitol, sorbitol, or sodium chloride in the composition. Pharmaceutically acceptable carriers or pharmaceutical carriers can further comprise minor amounts of auxiliary substances such as wetting or emulsifying agents, preservatives or buffers, which enhance the shelf life or effectiveness of the antioxidant compounds or of the pharmaceutical compositions containing same.

### Methods of the invention

The inventors have identified a link between the abundance of the six bacteria genera *Parabacteroides, Collinsella, Subdoligranulum, Anaerostipes, Akkermansia* and *Lactococcus* and the activity of the MS disease.

As a matter of fact, a high abundance of *Subdoligranulum*, *Anaerostipes*, *Akkermansia* and *Lactococcus* bacteria but low abundance of *Parabacteroides* and *Collinsella* bacteria highlights that the subject will experience a relapse very soon ("active" MS disease), whereas, on the contrary, a low abundance of *Subdoligranulum*, *Anaerostipes*, *Akkermansia* and *Lactococcus* bacteria but high abundance of *Parabacteroides* and *Collinsella* bacteria highlights that the subject is in a stable inactive MS state, and has low risk of experiencing a relapse in the coming days or weeks.

Accordingly, they propose to detect the expression or abundance level of any gene or specific fragment of said bacteria *Parabacteroides, Collinsella*, *Subdoligranulum*, *Anaerostipes*, *Akkermansia* and *Lactococcus,* in order to diagnose the susceptibility of a patient for a relapse or to determine the risk of said relapse in a short future (e.g., within one week).

The invention thus relates to a method for the *in vitro* diagnosis or prognosis of the activity of multiple sclerosis (MS) in a subject in need thereof, comprising determining from a stool sample from said subject a bacterial genus signature comprising or consisting of at least six genes or gene fragments, whose sequences are specific of the bacteria genera *Parabacteroides, Collinsella*, *Subdoligranulum*, *Anaerostipes*, *Akkermansia* and *Lactococcus.*

In other words, said signature comprises or consists of at least six different genes or gene fragments, each of them being specific for each of the six following genera: *Parabacteroides, Collinsella*, *Subdoligranulum*, *Anaerostipes*, *Akkermansia* and *Lactococcus.*

In a preferred embodiment, the method of the invention requires determining the abundance, preferably the relative abundance, of the bacteria genera *Parabacteroides, Collinsella*, *Subdoligranulum*, *Anaerostipes*, *Akkermansia* and *Lactococcus* in said sample, i.e., of the said at least six sequences.
This abundance profile can be assessed by measuring, as explained above, the absolute or relative abundance of:
(i) A representative gene or gene fragment of the bacteria genus *Parabacteroides,* or of a covariant gene or gene fragment thereof, and
(ii) A representative gene or gene fragment of the bacteria genus *Collinsella,* or of a covariant gene or gene fragment thereof, and
(iii) A representative gene or gene fragment of the bacteria genus *Subdoligranulum,* or of a covariant gene or gene fragment thereof, and
(iv) A representative gene or gene fragment of the bacteria genus *Anaerostipes,* or of a covariant gene or gene fragment thereof, and
(v) A representative gene or gene fragment of the bacteria genus *Akkermansia* or of a covariant gene or gene fragment thereof,
   and
(vi) A representative gene or gene fragment of the bacteria genus *Lactococcus*, or of a covariant gene or gene fragment thereof.

Some representative genes have been described above (SEQ ID NO:1-33). As explained, any covariant gene thereof can also be used.

In another embodiment, the invention also relates to an *in vitro* method for monitoring a MS treatment or for designing a treatment or for determining the responsiveness of a patient undergoing a relapse of multiple sclerosis to a particular treatment, said method comprising, as described above, determining from a stool sample from said subject a bacterial genus signature as described above.

Many of the disease-modifying therapies used to treat MS carry significant health risks. Selecting the right therapy will depend on careful consideration of many factors, including duration and severity of disease, effectiveness of previous MS treatments, other health issues, cost, and child-bearing status. The methods of the invention can trigger the decision to maintain / change a treatment or a dose thereof.

The invention also relates to a method for the *in vitro* diagnosis of multiple sclerosis (MS) in a subject in need thereof, comprising determining from a stool sample from said subject a bacterial genus signature comprising or consisting of at least six genes or gene fragments, whose sequences are specific of the bacteria genera *Parabacteroides, Collinsella*, *Subdoligranulum*, *Anaerostipes*, *Akkermansia* and *Lactococcus.* In this case, the score obtained from the tested patient will be compared to a reference score obtained from a healthy subject.

In another embodiment, the invention relates to an *in vitro* method for treating a patient suffering from MS, said method comprising, as described above, determining from a stool sample from said subject a bacterial genus signature as described above.

More preferably, in this embodiment, the invention encompasses a method for treating a subject suffering from MS, said method comprising the following steps:
i) diagnosing the activity of MS in a subject or his / her risk of experiencing a relapse according to the above-mentioned method,
   and
ii) treating said subject with an appropriate treatment, said appropriate treatment being chosen in those classically attributed by the practitioner once said state of MS is diagnosed.

For example, if a MS patient is diagnosed in an active state, an adapted treatment can be a combination of interferons with natalizumab (Tysabri®), a humanized monoclonal antibody which is quite effective but has been currently relegated to second-line based on concerns over life-threatening side effects. Also, corticosteroids (such as oral prednisone and intravenous methylprednisolone), can be prescribed to reduce nerve inflammation. Side effects may include insomnia, increased blood pressure, mood swings and fluid retention. Beta interferons and Glatiramer acetate (Copaxone, Glatopa) can also be prescribed. They are injected under the skin or into muscle and can reduce the frequency and severity of relapses. Side effects may include skin irritation at the injection site.

Oral treatments can also be given, such as Fingolimod (Gilenya), dimethyl fumarate (Tecfidera), Teriflunomide (Aubagio), and Siponimod (Mayzent).

Infusion treatment such as Ocrelizumab (Ocrevus), Natalizumab (Tysabri), Alemtuzumab (Campath, Lemtrada) and Mitoxantrone.

Ultimately, plasma exchange (plasmapheresis) can be performed. Accordingly, part of the patient's blood (plasma) is removed and separated from his blood cells. The blood cells are then mixed with a protein solution (albumin) and put back into his body.

Moreover, it is possible to use the methods of the invention for testing the efficiency of a treatment in a subject suffering from MS, or to evaluate the response of a patient to a treatment.

In this embodiment, the method of the invention comprises the following steps:
i) diagnosing the activity of MS before and after the administration of a treatment, according to the above-mentioned method,
   and
ii) concluding that the treatment is efficient in said subject if the state before the administration of the treatment was active but becomes inactive and stable upon administration of the treatment.

If the MS patient is diagnosed to be "active" before the administration of the treatment and becomes "stably inactive" upon administration of the treatment, then said patient is responding to said treatment. This efficient treatment should therefore be preferentially maintained.

Conversely, if the MS patient is diagnosed to be "active" before the administration of the treatment and remains "active" upon administration of the treatment, then said patient is not responding to said treatment, and it is better to replace said treatment with another one or to combine it with another treatment.

Of note, if the MS patient is diagnosed to be "stably inactive", then it is not necessary to administer a strong chemical treatment. An adapted treatment can then be physical therapy (stretching and strengthening exercises), muscle relaxants (such as baclofen (Lioresal) and tizanidine (Zanaflex)), amantadine (Gocovri, Oxmolex), modafinil (Provigil) and methylphenidate (Ritalin) that may be helpful in reducing MS-related fatigue. Some drugs used to treat depression, including selective serotonin reuptake inhibitors, may also be recommended. Specific diets, such as the Mediterranean diet, that improve the gut microbiome can also be recommended.

It is also recommended to provide the patient with individualized diets that specifically increase the levels of bacteria of the *Collinsella* and *Parabacteroides* genera for example by administering them a mixture of these bacteria, that will act as a probiotic drug. These bacteria are hereafter called "bacteria of the invention".

Accordingly, the present invention relates to a mixture of bacteria of the *Collinsella* and *Parabacteroides* genera, for use as a probiotic drug in patients suffering from a MS disease. This mixture can also contain a pharmaceutically acceptable carrier, as disclosed above. This drug can be used to prevent a MS relapse or treat a MS in patients in need thereof.

Preferably, said patients are those that have been diagnosed as having a MS or having possible a MS, according to the McDonald criteria revised in 2005 (Polman et al., 2006).

These pharmaceutical compositions may be in a variety of forms. These include, for example, liquid, semi-solid and solid dosage forms, such as liquid solutions (e.g., injectable and infusible solutions), dispersions or suspensions, tablets, pills, powders, liposomes and suppositories. In certain embodiments, the bacteria of the invention may be prepared with a carrier that will protect same against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are generally known to those skilled in the art.

These pharmaceutical compositions typically include a "therapeutically effective amount" or a "prophylactically effective amount" of the bacteria of the invention. A "therapeutically effective amount" refers to the amount of bacteria that is effective, at dosages and for periods of time necessary, to achieve the desired therapeutic result.

In a particular embodiment, the subject to be tested / treated by means of the methods of the invention is a human being afflicted with multiple sclerosis (MS). In another embodiment, the subject is a human being afflicted with a subtype of MS selected from: relapsing remitting MS (RRMS), primary progressive multiple sclerosis (PPMS) and secondary progressive multiple sclerosis (SPMS). In a particular embodiment, said subject is a patient who is at risk of a relapse. Each possibility is a separate embodiment.

The sample used in the methods of the invention is a stool sample. Indeed, such a sample may be obtained by a completely harmless collection from the patient. Preferably, said stool sample is collected and stored in appropriate buffers that do not denature or affect the DNA contained in same (in this aim, one can use, e.g., the DNA/RNA Shield® (Zymo Research), RNA later® RNA stabilization Reagent (Ambion), or the Stool DNA Stabilizer (Invitek), or a mix of EDTA and DMSO). More preferably, the samples are stored at -80°C until DNA extraction and subsequent analysis. Importantly, these samples are preferably whole stool samples, that has been obtained after classical defecation, in a sterile recipient.

Quantification of the microbial DNA present in the stool sample can be performed by any well-known method. The most commonly used methods known in the art for the quantification of DNA strands in a sample include Northern blotting and *in situ* hybridization (Parker & Barnes, 1999) and PCR-based methods, such as quantitative polymerase chain reaction (qPCR) (Heid et al., 1996). Alternatively, antibodies may be employed that can recognize sequence-specific duplexes, including DNA duplexes or DNA-protein duplexes. Representative methods for sequencing-based analysis include chain-termination methods, shotgun sequencing methods, de novo sequencing, next generation sequencing methods (including Massively Parallel Signature Sequencing (MPSS), Polony sequencing, 454 pyrosequencing, Illumina (Solexa) sequencing, SOLiD sequencing, Ion semiconductor sequencing, DNA nanoball sequencing, Helioscope single molecule sequencing, Single molecule real time (SMRT) sequencing, RNAP sequencing, Nanopore DNA sequencing, Sequencing by hybridization and Microfluidic Sanger sequencing).

It is also possible to measure bacterial DNA from a pool of DNA by i) sequencing the DNA present in stool samples using high throughput sequencing technologies and ii) by aligning the short reads obtained by means of these sequencing technologies to the bacterial genome or gene catalogs. In this case, "relative abundance of bacterial DNA" can be calculated by counting the number of reads mapped to a single reference sequence from the bacterial target (H) and the remaining number of reads generated (B), and normalizing the number of reads H by the total amount of reads (H+B).

In a particular embodiment, which should not be considered as limiting the scope of the invention, the determination of the bacterial genus profile using quantitative PCR may be performed as follows. Briefly, the real-time PCR reactions are carried out using the TaqMan Universal PCR Master Mix (Applied Biosystems). 6 µL cDNA is added to a 9 µL PCR mixture containing 7.5 µL TaqMan Universal PCR Master Mix, 0.75 µL of a 20X mixture of probe and primers and 0.75µl water. The reaction consisted of one initiating step of 2 min at 50 deg. C, followed by 10 min at 95 deg. C, and 40 cycles of amplification including 15 sec at 95 deg. C and 1 min at 60 deg. C. The reaction and data acquisition can be performed using the ABI 7900HT Fast Real-Time PCR System (Applied Biosystems)..

The amount of nucleic acid transcripts can be measured by any technology known by a man skilled in the art. In particular, the measure may be carried out directly on an extracted messenger RNA (mRNA) sample, or on retrotranscribed complementary DNA (cDNA) prepared from extracted mRNA by technologies well-known in the art. From the mRNA or cDNA sample, the amount of nucleic acid transcripts may be measured using any technology known by a man skilled in the art, including nucleic microarrays, quantitative PCR, digital PCR, and hybridization with a labelled probe.

In a particular embodiment, the methods of the invention may further comprise determining at least one additional parameter useful for the diagnosis. Such "parameters useful for the diagnosis" are parameters from the prior art that have been described as helpful to distinguish active from inactive MS patients (see e.g., WO 2010/033951, WO 2015/140793, and WO 2018/006051).

Such additional parameters may be used to confirm the diagnosis obtained using the expression profile of the invention.

The determination of the MS phenotype is carried out thanks to the calculation of a score that compilates the abundance of the at least six target genes. It is then compared to a reference score, in order to conclude that the patients suffer from a MS or an active MS or is at risk of a relapse.

A "reference score" is a predetermined score, obtained from a biological sample from a subject with a known particular response state (healthy, or MS patient in a define state). In order to have this comparison meaningful and robust, the "reference score" is determined by the training of an algorithm resulting from the following steps:
- the collection of the scores of biological samples obtained from active and inactive MS patients, or the collection of the scores of biological samples from healthy subjects,
- the training of an algorithm on the scores of the aforementioned patients for classifying said expression profiles as healthy, active and inactive MS phenotypes.

The comparison of a tested subject score with said reference score, which permits prediction of the tested subject's clinical response based on his/her expression profile, can be done by those skilled in the art using artificial intelligence, statistical models or machine learning technologies. The PLS (Partial Least Square) regression is particularly relevant to give prediction in the case of small reference samples. The comparison may also be performed using Support Vector Machines (SVM), logistic regression, Linear Discriminant Analysis, Random Forests, k-NN (Nearest Neighbour) or PAM (Predictive Analysis of Microarrays) statistical methods.

### Kits and arrays of the invention

In another preferred embodiment, the abundance or expression profile of the target bacteria of the invention is determined by the use of a nucleic microarray.

According to the invention, a "nucleic microarray" consists of different nucleic acid probes that are attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes can be nucleic acids such as cDNAs ("cDNA microarray") or oligonucleotides ("oligonucleotide microarray"), and the oligonucleotides may be about 25 to about 60 base pairs or less in length.

To determine the expression profile of a target nucleic sample, said sample is labelled, contacted with the microarray in hybridization conditions, leading to the formation of complexes between target nucleic acids that are complementary to probe sequences attached to the microarray surface. The presence of labelled hybridized complexes is then detected. Many variants of the microarray hybridization technology are available to the man skilled in the art.

In a preferred embodiment, the present invention relates to a nucleic microarray comprising or consisting of at least six oligonucleotides, whose sequences are complementary to at least six sequences that are specific for the bacteria genera *Parabacteroides, Collinsella*, *Subdoligranulum*, *Anaerostipes*, *Akkermansia* and *Lactococcus* respectively.

For example, it comprises or consists of oligonucleotides whose sequences are complementary to:
(i) the sequence of a representative fragment of the bacteria genus *Parabacteroides*, chosen among SEQ ID NO:1-8, or to a covariant sequence thereof, and
(ii) The sequence of a representative fragment of the bacteria genus *Collinsella*, chosen among SEQ ID NO:9-12, or to a covariant sequence thereof, and
(iii) The sequence of a representative fragment of the bacteria genus *Subdoligranulum*, of SEQ ID NO:13, or to a covariant sequence thereof, and
(iv) The sequence of a representative fragment of the bacteria genus *Anaerostipes*, chosen among the SEQ ID NO:14-19, or to a covariant sequence thereof, and
(v) The sequence of a representative fragment of the bacteria genus *Akkermansia*, SEQ ID NO:20, or to a covariant sequence thereof,
   and
(vi) The sequence of a representative fragment of the bacteria genus *Lactococcus,* chosen among the SEQ ID NO:21-33, or to a covariant sequence thereof,
and optionally one or more normalization gene(s).

Preferably, the oligonucleotides are about 50 bases in length.

Preferably, a nucleotide sequence specifically hybridizes to its complementary sequences only under stringent conditions. The parameters defining the stringency conditions depend on the temperature at which 50% of the paired strands separate (Tm). For sequences comprising more than 30 bases, Tm is defined by the relation: Tm = 81.5 + 0.11 (% G + C) + 16.6Log (concentration of cations) - 0.63 (% formamide) - (600 / number of bases) (Sambrook et al., 1989). For sequences of length less than 30 bases, Tm is defined by the relation: Tm = 4 (G + C) + 2 (A + T). Under appropriate stringency conditions, at which the aspecific sequences do not hybridize, the hybridization temperature is preferably 5 to 10°C below Tm, and the hybridization buffers used are preferably strong solutions having a high ionic strength. These conditions are preferably used in order to detect the hybridization of complementary sequences.

Suitable microarray oligonucleotides specific for any gene of the invention may be designed, based on the genomic sequence of each gene (see Table 2 Genbank accession numbers), using any method of microarray oligonucleotide design known in the art. In particular, any available software developed for the design of microarray oligonucleotides may be used, such as, for instance, the OligoArray software, the GoArrays software, the Array Designer software, the Primer3 software, or the Promide software.

In another embodiment, the expression profile is determined by the use of a protein microarray.

The invention further concerns a kit for the *in vitro* diagnosis of the activity of MS, or for the diagnosis of MS, said kit comprising at least six reagents that can specifically detect the presence of at least one gene or fragment of each of the six bacteria genera *Parabacteroides, Collinsella*, *Subdoligranulum*, *Anaerostipes*, *Akkermansia* and *Lactococcus.*

By "a reagent that can specifically detect the presence of the gene or fragment" is meant a reagent which specifically allows for the determination of the abundance or expression of said gene or fragment, i.e. a reagent specifically intended for the specific determination of the abundance or expression level of the genes or fragments of the signature of the invention. This definition excludes generic reagents useful for the determination of the abundance or expression level of any gene, such as Taq polymerase or an amplification buffer, although such reagents may also be included in a kit according to the invention.

In a preferred embodiment of a kit according to the invention, said kit is dedicated to the *in vitro* methods of the invention.

The kit of the invention preferably comprises no more than 50, 40, 30, 25, 20, preferably no more than 15, preferably no more than 10, preferably no more than 9, 8, 7, 6, 5, 4, 3, 2, or 1 reagent(s) for determining the abundance or expression level of a gene that does not belong to one of the above described (i) to (vi) and that is not a housekeeping gene.

Such a kit for the *in vitro* diagnosis of MS activity or of MS disease may further comprise instructions for determination of the presence or absence of a particular phenotype.

The kit of the invention preferably includes specific amplification primers and/or probes for the specific quantitative amplification of genes or fragments of (i) to (vi), and optionally one or more housekeeping gene(s), or the nucleic microarray mentioned above.

The determination of the expression profile may thus be performed using quantitative PCR and/or a nucleic microarray, preferably an oligonucleotide microarray, and/or protein microarrays.

In addition, the instructions for the determination of the MS activity preferably include at least one reference bacterial genus profile, or at least one reference sample for obtaining a reference bacterial genus profile. In a preferred embodiment, at least one reference bacterial genus profile is a MS active bacterial genus profile. Alternatively, at least one reference expression profile may be a MS inactive bacterial genus profile or a bacterial genus profile from a healthy subject. More preferably, the determination of the level of responsiveness is carried out by comparison with both MS active and MS inactive bacterial genus profiles as described above.

Having generally described this invention, a further understanding of characteristics and advantages of the invention can be obtained by reference to certain specific examples and figures which are provided herein for purposes of illustration only and are not intended to be limiting unless otherwise specified.

### EXAMPLES

### Example 1:

### Materials and Methods

Data Identification and Data Extraction : Gut microbiome studies were selected on the basis that they had been performed on Multiple Sclerosis (MS) Patients and compared to Healthy Controls. The technology used to study the microbiome had to be targeted gene sequencing also called "targeted metagenomics", in particular sequencing of hyper variable regions of the ribosomal RNA 16s gene. Additionally, the sequencing files (referred to as FASTQ files) had to be available on one of the following public data repositories: European Nucleotide Archive (EBI), Sequence Read Archive (NCBI) or DNA Bank of Japan (DDBJ). When necessary, the authors were contacted to clarify the data repository accession numbers. When replicate or longitudinal samples were available, only the earliest sample was selected.

Relevant studies were identified by performing searches on PubMed between 26/04/19 and 02/05/19 using the following keywords: "Multiple sclerosis microbiota".

Following those steps the five studies have been selected as they matched our research criteria : Cekanaviciute *et al.,* Chen *et al., Forbes et al., Jangi et al. and* Miyake *et al.*

**Table 1 : Technical and clinical information on the five selected studies.**

| | **Technical information** | | | **Clinical information** | |
|---|---|---|---|---|---|
| | **Region** | **Type** | **Platform** | **HC** | **MS** |
| **Cekanaviciute** ***et al**.* | V4 | Single-end | Illumina MiSeq | 71 | 71 |
| | | | | | |
| **Chen *et al*.** | V3-V5 | Paired-end | Illumina HiSeq 2000 | 36 | 31 |
| | | | | | |
| **Forbes *et al***. | V4 | Paired-end | Illumina MiSeq | 23 | 19 |
| **Jangi *et al.*** | V4 | Paired-end | Illumina MiSeq | 43 | 60 |
| | | | | | |
| | | | | | |
| **Miyake *et al.*** | V1-V2 | Single-end | Roche 454 | 40 | 20 |

The corresponding sequence data were downloaded from the data repositories using the following identifiers : ERP101460, PRJNA335855 , PRJNA450340 , PRJNA32105 and DRA000672, DRA000673, DRA000675, DRA000676, DRA000678-DRA000684, DRA002866-DRA002874, DRA002875-DRA002906, DRA000869-DRA000886.

All the five studies identified differentially abundant taxa between MS patients and healthy controls. Interestingly, however, none of the authors identified the same taxa, there was in fact extremely limited overlap between the five studies. This lack of overlap could be explained by many factors such as geographical differences, differences in the patient inclusion criteria (such as disease stage or treatment), and technological differences (from sample collection method through the DNA extraction protocol, the targeted regions, sequencing technology or the bioinformatics analytical pipeline).

To make the studies more comparable, so that a common MS signature of dysbiosis could be identified in a western population, we excluded the Japanese study by Miyake, reprocessed the original sequence files through a common bioinformatics pipeline and applied a robust analytical method, called meta-analysis (Ramasamy A et al, 2008).

### Data Quality and Processing :

All FASTQ files were processed using an internal bioinformatics pipeline which is based on Qiime2 (Bolyen et al, 2019) and uses RDP for taxonomic annotation. The pipeline was run manually, adapting for each study based on the hypervariable region being sequenced as well as the type of file, whether single end or paired end. Read counts were converted into percentages to adjust for individual differences in sequencing depth and then log transformed (log10). Samples that failed the quality control steps were excluded.

### Statistical Analysis :

The meta-analysis was performed on all five studies in RStudio, using the R package MicrobiomeR (BMC Biominformatics 2019) without variable adjustment at the genus taxonomical level.

To assess the performance of a diagnostic signature of MS, the six significant variables were combined using a logistic regression model and a cross validation approach (2/3 of the data were used as training set and 1/3 as the test set) for each study. The ROC curves were estimated using the AUC package (version 0.3.0).

### Results

The study by Cekanavicuite et al. was excluded a *posteriori* as the reprocessing of the data could not confirm the results reported in their publication. There were thus finally only three studies used in the meta-analysis, those were the studies by Chen et al., Forbes et al. and Jangi et al.

The following six bacterial genera were found to be significantly differentially abundant between MS and healthy control (HC): *Parabacteroides, Collinsella*, *Subdoligranulum*, *Anaerostipes*, *Akkermansia* and *Lactococcus.*

*Parabacteroides* and *Collinsella* were both decreased in MS patients while *Subdoligranulum*, *Anaerostipes*, *Akkermansia* and *Lactococcus* were increased.

Details can be found in Table 2.

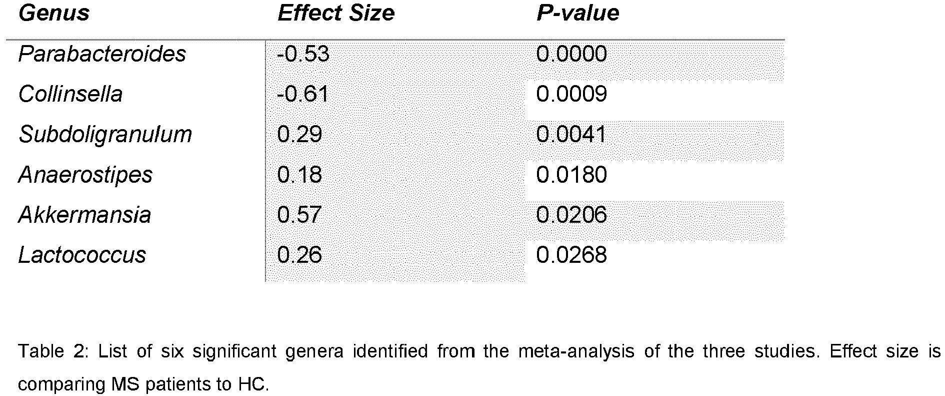

A signature combining the six bacterial genera led to AUC performances of 0.8 and 0.81 respectively on the studies by Forbes et al. and Chen et al. , and of 0.675 in the study by Jangi et al.

### BIBLIOGRAPHIC REFERENCES

Bolyen E, Rideout JR, Dillon MR, et al. 2019. Reproducible, interactive, scalable and extensible microbiome data science using QIIME 2. Nature Biotechnology 37: 852-857.
Cekanaviciute E, Yoo BB, Runia TF, Debelius JW, Singh S, Nelson CA, Kanner R, Bencosme Y, Lee YK, Hauser SL, Crabtree-Hartman E, Sand IK, Gacias M, Zhu Y, Casaccia P, Cree BAC, Knight R, Mazmanian SK, Baranzini SE. Gut bacteria from multiple sclerosis patients modulate human T cells and exacerbate symptoms in mouse models. Proc Natl Acad Sci U S A. 2017 Oct 3;114(40):10713-10718.
Chen J, Chia N, Kalari KR, Yao JZ, Novotna M, Paz Soldan MM, Luckey DH, Marietta EV, Jeraldo PR, Chen X, Weinshenker BG, Rodriguez M, Kantarci OH, Nelson H, Murray JA, Mangalam AK. Multiple sclerosis patients have a distinct gut microbiota compared to healthy controls. Sci Rep. 2016 Jun 27;6:28484.
Forbes JD, Chen CY, Knox NC, Marrie RA, El-Gabalawy H, de Kievit T, Alfa M, Bernstein CN, Van Domselaar G. A comparative study of the gut microbiota in immune-mediated inflammatory diseases-does a common dysbiosis exist? Microbiome. 2018 Dec 13;6(1):221.
Heid CA, Stevens J, Livak KJ, Williams PM. Real time quantitative PCR. Genome Research 1996 6:986-994.
Ho NT, Li F, Wang S, Kuhn L., MetamicrobiomeR: an R package for analysis of microbiome relative abundance data using zero-inflated beta GAMLSS and meta-analysis across studies using random effects models. BMC Bioinformatics. 2019 Apr 16;20(1): 188.
Jangi S, Gandhi R, Cox LM, Li N, von Glehn F, Yan R, Patel B, Mazzola MA, Liu S, Glanz BL, Cook S, Tankou S, Stuart F, Melo K, Nejad P, Smith K, Topçuolu BD, Holden J, Kivisakk P, Chitnis T, De Jager PL, Quintana FJ, Gerber GK, Bry L, Weiner HL. Alterations of the human gut microbiome in multiple sclerosis. Nat Commun. 2016 Jun 28;7:12015
Jovel J, Patterson J, Wang W, Hotte N, O'Keefe S, Mitchel T, Perry T, Kao D, Mason AL, Madsen KL, Wong GK (2016-01-01). Characterization of the Gut Microbiome Using 16S or Shotgun Metagenomics. Frontiers in Microbiology. 7: 459.
Miyake S, Kim S, Suda W, Oshima K, Nakamura M, Matsuoka T, Chihara N, Tomita A, Sato W, Kim SW, Morita H, Hattori M, Yamamura T. Dysbiosis in the Gut Microbiota of Patients with Multiple Sclerosis, with a Striking Depletion of Species Belonging to Clostridia XIVa and IV Clusters. PLoS One. 2015 Sep 14;10(9):e0137429
Parker & Barnes. mRNA: detection by in Situ and northern hybridization. Methods in Molecular Biology (1999) 106:247-283.
Pereira F, Carneiro J, Matthiesen R, van Asch B, Pinto N, Gusmão L, Amorim A (December 2010). Identification of species by multiplex analysis of variable-length sequences. Nucleic Acids Research. 38 (22): e203.
Polman et al., 2006, Revisions to the 2001 McDonald diagnostic criteria. Ann Neurol. ;59(4):727-8
Qin J, A metagenome-wide association study of gut microbiota in type 2 diabetes. Nature, 2012. Oct 4;490(7418):55-60.
Ramasamy A, Mondry A, Holmes CC, Altman DG. Key issues in conducting a meta-analysis of gene expression microarray datasets. PLoS Med. 2008 Sep 30;5(9):e184.
Schepici G. et al, 2019. The Gut Microbiota in Multiple Sclerosis: An Overview of Clinical Trials. Cell Transplant. 2019 Sep 12:963689719873890

## Claims

1. A method for the *in vitro* diagnosis or prognosis of the activity of multiple sclerosis (MS) in a subject in need thereof, comprising determining from a stool sample from said subject a bacterial genus signature comprising or consisting of at least six genes or gene fragments, whose sequences are specific for the bacteria genera *Parabacteroides, Collinsella*, *Subdoligranulum*, *Anaerostipes*, *Akkermansia* and *Lactococcus.*

2. The method according to claim 1, wherein said signature comprises:
(i) A representative gene fragment of the bacterial genus *Parabacteroides,* or a covariant thereof, and
(ii) A representative gene fragment of the bacterial genus *Collinsella,* or a covariant thereof, and
(iii) A representative gene fragment of the bacterial genus *Subdoligranulum,* or a covariant thereof, and
(iv) A representative gene fragment of the bacterial genus *Anaerostipes,* or a covariant thereof, and
(v) A representative gene fragment of the bacterial genus *Akkermansia* or a covariant thereof, and
(vi) A representative gene fragment of the bacterial genus *Lactococcus,* or a covariant thereof.

3. The method according to claim 2, wherein the sequence of said representative gene fragment (i) is chosen in SEQ ID NO:1-8.

4. The method according to claim 2 or 3, wherein the sequence of said representative gene fragment (ii) is chosen in SEQ ID NO:9-12.

5. The method according to any of claims 2 to 4, wherein the sequence of said representative gene fragment (iii) is SEQ ID NO:13.

6. The method according to any of claims 2 to 5, wherein the sequence of said representative gene fragment (iv) is chosen in SEQ ID NO:14-19.

7. The method according to any of claims 2 to 6, wherein the sequence of said representative gene fragment (v) is SEQ ID NO:20.

8. The method according to any of claims 2 to 7, wherein the sequence of said representative gene fragment (v) is chosen in SEQ ID NO:21-33.

9. The method according to any of claims 1-8, comprising determining the abundance, preferably the relative abundance, of said at least six sequences in said stool sample.

10. An *in vitro* method for monitoring a treatment or for designing a treatment or for determining the responsiveness of a patient undergoing a relapse of multiple sclerosis to a particular treatment, said method comprising determining from a stool sample from said subject, a bacterial genus signature as described in any of claims 1-8.

11. An *in vitro* method for testing the efficiency of a treatment in a subject suffering from MS, or to evaluate the response of a patient to a treatment, said method comprising :
i) diagnosing the activity of MS before and after the administration of a treatment, by determining a bacterial genus signature as described in any of claims 1-8 and
ii) concluding that the treatment is efficient in said subject if the state before the administration of the treatment was active but becomes inactive and stable upon administration of the treatment.

12. The method of any of claims 1-11, wherein said subject is a human being that is at risk of a MS relapse.

13. A nucleic microarray comprising or consisting of at least six oligonucleotides, whose sequences are complementary to at least six sequences that are specific for the bacteria genera *Parabacteroides, Collinsella*, *Subdoligranulum*, *Anaerostipes*, *Akkermansia* and *Lactococcus* respectively.

14. The nuclei microarray of claim 13, wherein said at least six sequences are chosen from SEQ ID NO:1-33.

15. A mixture of bacteria of the *Collinsella* and *Parabacteroides* genera for use as a probiotic drug in patients suffering from a multiple sclerosis disease.


SEQUENCE LISTING

<110>  LUXIA SCIENTIFIC

<120>  Bacterial combinations predictive of the activity of Multiple
       Sclerosis

<130>  B75498EP - D39793 - GFA

<160>  33

<170>  PatentIn version 3.5

<210>  1
<211>  1492
<212>  DNA
<213>  Parabacteroides


<220>
<221>  misc_feature
<223>  AB261128  _ S000727759

<400>  1
agagtttgat cctggctcag gatgaacgct agcgacaggc ttaacacatg caagtcgagg      60

ggcagcatgg taagtagcaa tacttattga tggcgaccgg cgcacgggtg agtaacgcgt     120

atgcaactta cctatcagag ggggatagcc cggcgaaagt cggattaata ctccataaaa     180

caggggttcc gcatgggact atttgttaaa gattcatcgc tgatagatag gcatgcgttc     240

cattaggcag ttggcggggt aacggcccac caaaccgacg atggataggg gttctgagag     300

gaaggtcccc cacattggta ctgagacacg gaccaaactc ctacgggagg cagcagtgag     360

gaatattggt caatggccga gaggctgaac cagccaagtc gcgtgaagga tgaaggatct     420

atggtttgta aacttctttt ataggggaat aaagtgtggg acgtgttcca ttttgtatgt     480

accctatgaa taagcatcgg ctaactccgt gccagcagcc gcggtaatac ggaggatgcg     540

agcgttatcc ggatttattg ggtttaaagg gtgcgtaggt ggtaatttaa gtcagcggtg     600

aaagtttgtg gctcaaccat aaaattgccg ttgaaactgg gttacttgag tgtgtttgag     660

gtaggcggaa tgcgtggtgt agcggtgaaa tgcatagata tcacgcagaa ctccaattgc     720

gaaggcagct tactaaacca taactgacac tgaagcacga aagcgtgggt atcaaacagg     780

attagatacc ctggtagtcc acgcagtaaa cgatgattac taggagtttg cgatacacag     840

taagctctac agcgaaagcg ttaagtaatc cacctgggga gtacgccggc aacggtgaaa     900

ctcaaaggaa ttgacggggg cccgcacaag cggaggaaca tgtggtttaa ttcgatgata     960

cgcgaggaac cttacccggg tttgaacgta gtcagaccga ccttgaaaga ggtcttctag    1020

caatagctga ttacgaggtg ctgcatggtt gtcgtcagct cgtgccgtga ggtgtcggct    1080

taagtgccat aacgagcgca acccttatca ctagttacta acaggtcaag ctgaggactc    1140

tggtgagact gccagcgtaa gctgtgagga aggtggggat gacgtcaaat cagcacggcc    1200

cttacatccg gggcgacaca cgtgttacaa tggcatggac aaagggcagc tacctggcga 1260

caggatgcta atctctaaac catgtctcag ttcggatcgg agtctgcaac tcgactccgt 1320

gaagctggat tcgctagtaa tcgcgcatca gccatggcgc ggtgaatacg ttcccgggcc 1380

ttgtacacac cgcccgtcaa gccatgggag ccgggggtac ctgaagtccg taaccgcaag 1440

gatcggccta gggtaaaact ggtgactggg gctaagtcgt aacaaggtaa cc 1492

<210> 2
<211> 1396
<212> DNA
<213> Parabacteroides

<220>
<221> misc_feature
<223> AY974070 S000577391

<400> 2
gtcgaggggc agcacggatg tagcaataca ttggtggcga ccggcgcacg ggtgagtaac 60

gcgtatgcaa cctgcctatc agaggggaat aacccggcga aagtcggact aataccgcat 120

aaaacagggg ttccacatgg aaatatttgt taaagaatta tcgctgatag atgggcatgc 180

gttccattag atagttggtg aggtaacggc tcaccaagtc cacgatggat aggggttctg 240

agaggaaggt cccccacact ggtactgaga cacggaccag actcctacgg gaggcagcag 300

tgaggaatat tggtcaatgg gcgagagcct gaaccagcca agtcgcgtga aggatgaagg 360

atctatggtt tgtaaacttc ttttatatgg gaataaagtg aggaacgtgt tcctttttgt 420

atgtaccata tgaataagca tcggctaact ccgtgccagc agccgcggta atacggagga 480

tgcgagcgtt atccggattt attgggttta aagggtgcgt aggtggttaa ttaagtcagc 540

ggtgaaagtt tgtggctcaa ccataaaatt gccgttgaaa ctggttgact tgagtatatt 600

tgaggtaggc ggaatgcgtg gtgtagcggt gaaatgcata gatatcacgc agaactccga 660

ttgcgaaggc agcttactaa actataactg acactgaagc acgaaagcgt ggggatcaaa 720

caggattaga taccctggta gtccacgcag taaacgatga ttactagctg tttgcgatac 780

acagtaagcg gcacagcgaa agcgttaagt aatccacctg gggagtacgc cggcaacggt 840

gaaactcaaa ggaattgacg ggggcccgca caagcggagg aacatgtggt ttaattcgat 900

gatacgcgag gaaccttacc cgggtttgaa cgcattatga cagctctgga aacacattct 960

ctagtaatag caatttgcga ggtgctgcat ggttgtcgtc agctcgtgcc gtgaggtgtc 1020

ggcttaagtg ccataacgag cgcaaccctt atcactagtt actaacaggt caagctgagg 1080

actctagtga gactgccagc gtaagctgtg aggaaggtgg ggatgacgtc aaatcagcac 1140

ggcccttaca tccggggcga cacacgtgtt acaatggtgg ggacaaaggg cagctacctg 1200

gcgacaggat gctaatctcc aaaccccatc tcagttcgga tcgaagtctg caacccgact 1260

tcgtgaagct ggattcgcta gtaatcgcgc atcagccatg gcgcggtgaa tacgttcccg 1320

ggccttgtac acaccgcccg tcaagccatg ggagttgggg gtacctaaag tccgtaaccg 1380

caaggatcgg cctagg 1396

<210> 3
<211> 1488
<212> DNA
<213> Parabacteroides

<220>
<221> misc_feature
<223> AB238922 S000650586

<400> 3
agagtttgat cctggctcag gatgaacgct agcgacaggc ttaacacatg caagtcgagg 60

ggcagcgggg tgtagcaata caccgccggc gaccggcgca cgggtgagta acgcgtatgc 120

aacttgccta tcagaggggg ataacccggc gaaagtcgga ctaataccgc atgaagcagg 180

gatcccgcat gggaatattt gctaaagatt catcgctgat agataggcat gcgttccatt 240

aggcagttgg cggggtaacg gcccaccaaa ccgacgatgg ataggggttc tgagaggaag 300

gtcccccaca ttggtactga gacacggacc aaactcctac gggaggcagc agtgaggaat 360

attggtcaat gggcgtaagc ctgaaccagc caagtcgcgt gagggatgaa ggttctatgg 420

atcgtaaacc tcttttataa gggaataaag tgcgggacgt gtcccgtttt gtatgtacct 480

tatgaataag gatcggctaa ctccgtgcca gcagccgcgg taatacggag gatccgagcg 540

ttatccggat ttattgggtt taaagggtgc gtaggcggcc ttttaagtca gcggtgaaag 600

tctgtggctc aaccatagaa ttgccgttga aactgggggg cttgagtatg tttgaggcag 660

gcggaatgcg tggtgtagcg gtgaaatgca tagatatcac gcagaacccc gattgcgaag 720

gcagcctgcc aagccattac tgacgctgat gcacgaaagc gtggggatca aacaggatta 780

gataccctgg tagtccacgc agtaaacgat gatcactagc tgtttgcgat acactgtaag 840

cggcacagcg aaagcgttaa gtgatccacc tggggagtac gccggcaacg gtgaaactca 900

aaggaattga cgggggcccg cacaagcgga ggaacatgtg gtttaattcg atgatacgcg 960

aggaacctta cccgggtttg aacgcattcg gaccgaggtg gaaacacctt ttctagcaat 1020

agccgtttgc gaggtgctgc atggttgtcg tcagctcgtg ccgtgaggtg tcggcttaag 1080

tgccataacg agcgcaaccc ttgccactag ttactaacag gttaggctga ggactctggt 1140

gggactgcca gcgtaagctg cgaggaaggc ggggatgacg tcaaatcagc acggccctta 1200

catccggggc gacacacgtg ttacaatggc gtggacaaag ggaggccacc tggcgacagg 1260

gagcgaatcc ccaaaccacg tctcagttcg gatcggagtc tgcaacccga ctccgtgaag 1320

ctggattcgc tagtaatcgc gcatcagcca tggcgcggtg aatacgttcc cgggccttgt 1380

acacaccgcc cgtcaagcca tgggagccgg gggtacctga agtccgtaac cgaaaggatc 1440

ggcctagggt aaaactggtg actggggcta agtcgtaaca aggtaacc 1488

<210> 4
<211> 1489
<212> DNA
<213> Parabacteroides

<220>
<221> misc_feature
<223> AB574482 S003620294

<400> 4
agagtttgat cctggctcag gatgaacgct agcgacaggc ttaacacatg caagtcgagg 60

ggtagcaggg ggtagcaata tcccgctgac gaccggcgca cgggtgagta acgcgtatgc 120

aacttaccta tcagagaggg ataacccggc gaaagtcgga ctaataccac atgaaacagg 180

ggtcccgcat gggaatattt gttaaagatt tattgctgat agataggcat gcgttccatt 240

agatagttgg tggggtaacg gcccaccaag tctgcgatgg ataggggttc tgagaggaag 300

gtcccccaca ttggtactga gacacggacc aaactcctac gggaggcagc agtgaggaat 360

attggtcaat gggcgagagc ctgaaccagc caagtcgcgt gaaggatgaa ggttctatgg 420

attgtaaact tcttttagag gggaataaag tgcgggacgt gtcctgtttt gtatgtaccc 480

tctgaataag catcggctaa ctccgtgcca gcagccgcgg taatacggag gatgcgagcg 540

ttatccggat ttattgggtt taaagggtgc gtaggtggat gtttaagtca gcggtgaaag 600

tttgtggctc aaccataaaa ttgccgttga aactggatat cttgagtatg tttgaggtag 660

gcggaatgcg tggtgtagcg gtgaaatgca tagatatcac gcagaactcc gattgcgaag 720

gcagcttact aaaccaacac tgacactgaa gcacgaaagc gtgggtatca aacaggatta 780

gataccctgg tagtccacgc agtaaacgat gattactagg agtttgcgat atattgtaag 840

ctctacagcg aaagcgttaa gtaatccacc tggggagtac gccggcaacg gtgaaactca 900

aaggaattga cgggggcccg cacaagcgga ggaacatgtg gtttaattcg atgatacgcg 960

aggaacctta cccgggtttg aacgtaagaa gaccggagtg gaaacactct ttctagcaat 1020

agctttttac gaggtgctgc atggttgtcg tcagctcgtg ccgtgaggtg tcggcttaag 1080

tgccataacg agcgcaaccc ttatcactag ttactaacag gtcaagctga ggactctagt 1140

gagactgcca gcgtaagctg tgaggaaggt ggggatgacg tcaaatcagc acggccctta 1200

catccggggc gacacacgtg ttacaatggc atggacaaag ggcggctaca cagcgatgtg 1260

atgctaatcc ccaaaccatg tctcagttcg gatcggagtc tgcaactcga ctccgtgaag 1320

ctggattcgc tagtaatcgc gcatcagcca tggcgcggtg aatacgttcc cgggccttgt 1380

acacaccgcc cgtcaagcca tgggagccgg gggtacctga agtccgtaac cgttgaggat 1440

cggcctaggg taaaactggt gactggggct aagtcgtaac aaggtaacc 1489

<210> 5
<211> 1485
<212> DNA
<213> Parabacteroides

<220>
<221> misc_feature
<223> AB470343 S001291847

<400> 5
agagtttgat cctggctcag gatgaacgct agcgacaggc ttaacacatg caagtcgagg 60

ggcagcagga agtagcaata ctttgctggc gaccggcgca cgggtgagta acgcgtatgc 120

aacctaccta tcagaggggg ataacccggc gaaagtcgga ctaataccgc ataaaacagg 180

ggtcccgcat gggaatattt gttaaagatt aattgctgat agatgggcat gcgttccatt 240

agatagttgg taaggtaacg gcttaccaag tctgcgatgg ataggggttc tgagaggaag 300

gtcccccaca ctggtactga gacacggacc agactcctac gggaggcagc agtgaggaat 360

attggtcaat gggcgagagc ctgaaccagc caagtcgcgt gaaggatgaa ggatctatgg 420

ttcgtaaact tcttttataa gggaataaag tgcggacgtg tcctgttttg tatgtacctt 480

atgaataagg atcggctaac tccgtgccag cagccgcggt aatacggagg atccgagcgt 540

tatccggatt tattgggttt aaagggtgcg taggtggttt attaagtcag cggtgaaagt 600

ttgtggctca accataaaat tgccgttgaa actggttaac ttgagtatat ttgaggtagg 660

cggaatgcgt ggtgtagcgg tgaaatgcat agatatcacg cagaactcca attgcgaagg 720

cagcttacta aactataact gacactgaag cacgaaagcg tggggatcaa acaggattag 780

ataccctggt agtccacgca gtaaacgatg attactagga gtttgcgata cacagtaagc 840

tctacagcga aagcgttaag taatccacct ggggagtacg ccggcaacgg tgaaactcaa 900

aggaattgac gggggcccgc acaagcggag gaacatgtgg tttaattcga tgatacgcga 960

ggaaccttac ccgggtttga acgcattgga cagtccttga aagaggatct ctagcaatag 1020

ccatttgcga ggtgctgcat ggttgtcgtc agctcgtgcc gtgaggtgtc ggcttaagtg 1080

ccataacgag cgcaaccctt atctttagtt actaacaggt ctgctgagga ctctaaagag 1140

actgccagcg taagctgtga ggaaggtggg gatgacgtca aatcagcacg gcccttacat 1200

ccggggcgac acacgtgtta caatggtggg gacaaagggc agctacacag cgatgtgatg 1260

ctaatctcca aaccccatct cagttcggat cgaagtctgc aacccgactt cgtgaagctg 1320

gattcgctag taatcgcgca tcagccatgg cgcggtgaat acgttcccgg gccttgtaca 1380

caccgcccgt caagccatgg gagttggggg tacctaaagt ccgtaaccgc aaggatcggc 1440

ctaggtaaaa ccgatgactg gggctaagtc gtaaccaagg taacc 1485

<210> 6
<211> 1492
<212> DNA
<213> parabacteroides

<220>
<221> misc_feature
<223> AB238928 S000650592

<220>
<221> misc_feature
<223> AB238928 S000650592

<400> 6
agagtttgat cctggctcag gatgaacgct agcgacaggc ttaacacatg caagtcgagg 60

ggcagcatga tttgtagcaa tacagattga tggcgaccgg cgcacgggtg agtaacgcgt 120

atgcaactta cctatcagag ggggatagcc cggcgaaagt cggattaata ccccataaaa 180

caggggtccc gcatgggaat atttgttaaa gattcatcgc tgatagatag gcatgcgttc 240

cattaggcag ttggcggggt aacggcccac caaaccgacg atggataggg gttctgagag 300

gaaggtcccc cacattggta ctgagacacg gaccaaactc ctacgggagg cagcagtgag 360

gaatattggt caatggccga gaggctgaac cagccaagtc gcgtgaagga agaaggatct 420

atggtttgta aacttctttt ataggggaat aaagtggagg acgtgtcctt ttttgtatgt 480

accctatgaa taagcatcgg ctaactccgt gccagcagcc gcggtaatac ggaggatgcg 540

agcgttatcc ggatttattg ggtttaaagg gtgcgtaggt ggtgatttaa gtcagcggtg 600

aaagtttgtg gctcaaccat aaaattgccg ttgaaactgg gttacttgag tgtgtttgag 660

gtaggcggaa tgcgtggtgt agcggtgaaa tgcatagata tcacgcagaa ctccgattgc 720

gaaggcagct tactaaacca taactgacac tgaagcacga aagcgtgggg atcaaacagg 780

attagatacc ctggtagtcc acgcagtaaa cgatgattac taggagtttg cgatacaatg 840

taagctctac agcgaaagcg ttaagtaatc cacctgggga gtacgccggc aacggtgaaa 900

ctcaaaggaa ttgacggggg cccgcacaag cggaggaaca tgtggtttaa ttcgatgata 960

cgcgaggaac cttacccggg tttgaacgta gtctgaccgg agtggaaaca ctccttctag 1020

caatagcaga ttacgaggtg ctgcatggtt gtcgtcagct cgtgccgtga ggtgtcggct 1080

taagtgccat aacgagcgca acccttatca ctagttacta acaggtgaag ctgaggactc 1140

tggtgagact gccagcgtaa gctgtgagga aggtggggat gacgtcaaat cagcacggcc 1200

cttacatccg gggcgacaca cgtgttacaa tggcatggac aaagggcagc tacctggcga 1260

caggatgcta atctccaaac catgtctcag ttcggatcgg agtctgcaac tcgactccgt 1320

gaagctggat tcgctagtaa tcgcgcatca gccatggcgc ggtgaatacg ttcccgggcc 1380

ttgtacacac cgcccgtcaa gccatgggag ccgggggtac ctgaagtccg taaccgcaag 1440

gatcggccta gggtaaaact ggtgactggg gctaagtcgt aacaaggtaa cc 1492

<210> 7
<211> 1442
<212> DNA
<213> parabacteroides

<220>
<221> misc_feature
<223> AB739697 S003286972

<400> 7
atagtttgat cctggctcag gatgaacgct agcgacaggc ttaacacatg caagtcgagg 60

ggcagcgggg ggtagcaata ctcygccggc gaccggcgca cgggtgagta acgcgtatgc 120

aacctaccta tcagagggga ataacccggc gaaagtcgga ctaataccgc ataaaacagg 180

ggtcccgcat ggggatattt gttaaagatt tatcgctgat agatgggcat gcgttccatt 240

agatagttgg tgaggtaacg gctcaccaag tctacgatgg ataggggttc tgagaggaag 300

gtcccccaca ctggtactga gacacggacc agactcctac gggaggcagc agtgaggaat 360

attggtcaat ggrcgagagt ctgaaccagc caagtcgcgt gaaggatgaa ggatctatgg 420

ttcgtaaact tcttttatag gggaataaag tgggagacgt gtcctgtttt gtatgtaccc 480

tatgaataag gatcggctaa ctccgtgcca gcacccgcgg taatacagaa gatccgagcg 540

ttatccggat ttattgggtt taaagggtgc gtaggtggtt ttttaagtca gcggtgaaag 600

tttgtggctc aaccataaaa ttgccgttga aactggaaga cttgagtatg tttgaggtag 660

gcggaatgcg tggtgtagcg gtgaaatgca tagatatcac gcagaactcc aattgcgaag 720

gcagcttact aaaccataac tgacactgaa gcacgaaagc gtggggatca aacaggatta 780

gataccctgg tagtccacgc agtaaacgat gattactagc tgtttgcgat aaacggtaag 840

cggcacagcg aaagcgttaa gtaatccacc tggggagtac gccggcaacg gtgaaactca 900

aaggaattga cgggggcccg cacaagcgga ggaacatgtg gtttaattcg atgatacgcg 960

aggaacctta cccgggtttg aacgcatttg gaccggactg gaaacagtyy ttctagcaat 1020

agccatttgc gaggtgctgc atggttgtcg tcagctcgtg ccgtgaggtg tcggcttaag 1080

tgccataacg agcgcaaccc ttatctttag ttactaacag gtgatgctga ggactctaaa 1140

gagactgcca gcgtaagctg tgaggaaggt ggggatgacg tcaaatcagc acggccctta 1200

catccggggc gacacacgtg ttacaatggc atggacaaag ggcagctacc tggcgacagg 1260

atgctaatct ctaaaccatg tctcagttcg gatcgaagtc tgcaacccga cttcgtgaag 1320

ctggattcgc tagtaatcgc gcatcagcca tggcgcggtg aatacgttcc cgggccttgt 1380

acacaccgcc cgtcaagcca tgggagttgg gggtacctaa agtccgtaac cgcaaggatc 1440

gg 1442

<210> 8
<211> 1481
<212> DNA
<213> parabacteroides

<220>
<221> misc_feature
<223> JN029805 S002915318

<400> 8
agagtttgat cctggctcag gatgaacgct agcgacaggc ttaacacatg caagtcgagg 60

ggcagcatag aagtagcaat actttggtgg cgaccggcgc acgggtgagt aacgcgtatg 120

caacctacct atcagagggg aataacccgg cgaaagtcgg actaataccg cataaaacag 180

gggcaccgca tggtgatatt tgttaaagaa attcgctgat agatgggcat gcgttccatt 240

aggtagttgg tgaggtaacg gctcaccaag ccgacgatgg ataggggaac tgagaggttg 300

gtcccccaca ctggtactga gacacggacc agactcctac gggaggcagc agtgaggaat 360

attggtcaat gggcgggagc ctgaaccagc caagtcgcgt gaaggaagaa ggatctatgg 420

ttcgtaaact tcttttgcag gggaataaag tgcgggacgt gtcctgtttt gtatgtaccc 480

tgagaataag gatcggctaa ctccgtgcca gcagccgcgg taatacggag gatccgagcg 540

ttatccggat ttattgggtt taaagggtgc gtaggtggtt tgataagtca gcggtgaaag 600

tttgcagctt aactgtaaaa atgccgttga aactgtcgga cttgagtgta aatgaggtag 660

gcggaatgcg tggtgtagcg gtgaaatgca tagatatcac gcagaactcc gattgcgaag 720

gcagcttact aagctacaac tgacactgaa gcacgaaagc gtggggatca aacaggatta 780

gataccctgg tagtccacgc agtaaacgat gattactagc tgtttgcgat acacagtaag 840

cggcacagcg aaagcgttaa gtaatccacc tggggagtac gccggcaacg gtgaaactca 900

aaggaattga cgggggcccg cacaagcgga ggaacatgtg gtttaattcg atgatacgcg 960

aggaacctta cccgggtttg aacgtaggga gacaggggtg gaaacatcct tttcagcaat 1020

gactgcctac gaggtgctgc atggttgtcg tcagctcgtg ccgtgaggtg tcggcttaag 1080

tgccataacg agcgcaaccc ttatttccag ttactaacag gtgatgctga ggactctgga 1140

gagactgcca gcgtaagctg tgaggaaggt ggggatgacg tcaaatcagc acggccctta 1200

catccggggc gacacacgtg ttacaatggt aaggacaaag ggcagctacc gggcgaccgg 1260

atgcgaatct ctaaacctta tctcagttcg gatcggagtc tgcaactcga ctccgtgaag 1320

ctggattcgc tagtaatcgc gcatcagcca tggcgcggtg aatacgttcc cgggccttgt 1380

acacaccgcc cgtcaagcca tgggagccgg gggtacctga agttcgtaac cgtaaggatc 1440

gacctagggt aaaactggtg actggggcta agtcgtaaca a 1481

<210> 9
<211> 1459
<212> DNA
<213> Collinsella

<220>
<221> misc_feature
<223> AB031063 S000382905

<400> 9
agagtttgat cctggctcag gatgaatgct ggcggcgcgc ctaacacatg caagtcgaac 60

ggcaccaccc tcgggtggaa gcaagtggcg aacggctgag taacacgtgg agaacccgcc 120

ccctcctccg ggatagcctc gggaaaccgt gggtaatacc ggatgacccc gcagggccgc 180

atggccctgc ggcaaagccc agacgggagg ggatgctccg cggcccatca ggtagacggc 240

ggggtgacgg cccaccgtgc cgacgacggg tagccgggtt gagagaccga ccggccagat 300

tgggactgag acacggccca gactcctacg ggaggcagca gtggggaatc ttgcgcaatg 360

gggggaaccc tgacgcagcg acgccgcgtg cgggacgaag gccctcgggt cgtaaaccgc 420

tttcagcagg gatgagacaa gacggtacct gcagaagaag ccccggctaa ctacgtgcca 480

gcagccgcgg taatacgtag ggggcgaccg ttatccggat tcattgggcg taaagcgcgc 540

gtaggcggcc cggcaggcag ggggtcaaat ggcggggctc aaccccgtcc cgccccctga 600

accgccgggc tcgggtccgg taggggaggg tggaacaccc ggtgtagcgg tggaatgcgc 660

agatatcggg tggaacaccg gtggcgaagg ggcctctggg ccgagaccga cgctgaggcg 720

cgaaagctgg gggagcgaac aggattagat accctggtag tcccagccgt aaacgatgga 780

cgctaggtgt ggggggacga tccctccgtg ccgcagccaa cgcattaacg tcccgcctgg 840

gaagtacggc cgcaaggcta aaactcaaag gaattgacgg gggcccgcac aagcagcgga 900

gcatgtggct taattcgaag caacgcgaag aaccttacca gggcttgaca tgaccgtgaa 960

gccgggaaga cccggtggcc gagaggagcg gtcacaggtg gtgcaatggc tgtcgtcagc 1020

tcgtgtcgtg agatgtttgg ttaagtccag caacgagcgc aacccccgcc gcatgttgcc 1080

agcacctcgg gtgggcaccc atgcgggacc gccggagtca atccggagga gggcggggac 1140

gacgtcaagt catcatgccc cttatgccct gggctgcaca cgtgctacaa tggccggtac 1200

agcgggatgc gatggcgcga gcggagcgga tccctgaaag ccggccccag ttcggattgg 1260

gggctgcaac ccgcccccat gaagtcggag ttgctagtaa tcgcggatca gcatgccgcg 1320

gtgaatgcgt tcccgggcct tgtacacacc gcccgtcaca ccacccgagt cgtctgcacc 1380

cgaagtcgcc ggcccaacca tctgggggga ggcgcctaag tatagagggt gaggtgggtg 1440

aagtcgtaac aaggtaacc 1459

<210> 10
<211> 1491
<212> DNA
<213> Collinsella

<220>
<221> misc_feature
<223> AB490807 S001587406

<400> 10
tcatggctca ggatgaacgc tggcggcgcg cctaacacat gcaagtcgaa cggcacccct 60

cctcggaggg aagcgagtgg cgaacggctg agtaacacgt ggagaacctg ccccctcctc 120

ggggatagcc gcgggaaacc gcgggtaaca cccgacgacc ccggagccgc gcatgcggcg 180

ccgggcaaat ctccggaggg aggggatggc tccgcggccc atcaggtaga cggcggggta 240

acggcccacc gtgccgacaa cgggtagccg ggttgagaga ccgaccggcc agattgggac 300

tgagacacgg cccagactcc tacgggaggc agcagtgggg aatcttgcgc aatgggggca 360

accctgacgc agcgacgccg cgtgcgggac gaaggccttc gggtcgtaaa ccgctttcag 420

cagggacgag acaagacggt acctgcagaa gaagccccgg ctaactacgt gccagcagcc 480

gcggtaatac gtagggggcg agcgttatcc ggattcattg ggcgtaaagc gcgcgtaggc 540

ggccgcgtag gcggggggtc aaatcccggg gctcaacccc ggtccgcccc ccgaaccccg 600

cggctcgggt ccggtagggg agggtggaat tcccggtgta gcggtggaat gcgcagatat 660

cgggaggaac accggtggcg aaggcggccc tctgggccga gaccgacgct gaggcgcgaa 720

agctggggga gcgaacagga ttagataccc tggtagtccc agccgtaaac gatggacgct 780

aggtgtgggg ggacgatccc tccgtgccgc agccaacgca ttaagcgtcc cgcctgggga 840

gtacggccgc aaggctaaaa ctcaaaggaa ttgacggggg cccgcacaag cagcggagca 900

tgtggcttaa ttcgaagcaa cgcgaagaac cttaccaggg cttgacatgt gcgtgaagcc 960

ggggagaccc cggtggccga gaggagcgca cacaggtggt gcatggctgt cgtcagctcg 1020

tgtcgtgaga tgttgggtta agtcccgcaa cgagcgcaac ccccgccgca tgttgccagc 1080

aggtcaggct gggcacccat gcgggaccgc cggcgtcaag ccggaggagg gcggggacga 1140

cgtcaagtca tcatgcccct tatgccctgg gctgcacacg tgctacaatg gccggtacag 1200

cgggatgcga tggcgcgagc cggagcggat ccctcaaagc cggccccagt tcggattggg 1260

ggctgcaacc cgcccccatg aagtcggagt tgctagtaat cgcggatcag catgccgcgg 1320

tgaatgcgtt cccgggcctt gtacacaccg cccgtcacac cacccgagtc gtctgcaccc 1380

gaagccgccg gcccaacctt ccgagggggg aggcgtcgaa ggtgtggagg gtgagggggg 1440

tgaagtcgta acaaggtagc cgtacgggaa cgtgcggctg gatcacctcc t 1491

<210> 11
<211> 1463
<212> DNA
<213> Collinsella

<220>
<221> misc_feature
<223> AB011816 S000382127

<220>
<221> misc_feature
<222> (862)..(862)
<223> n is a, c, g, or t

<400> 11
agagtttgat cctggctcag gatgaacgct ggcggcgcgc ctaacacatg caagtcgaac 60

ggcacccctc tccggaggga agcgagtggc gaacggctga gtaacacgtg gagaacctgc 120

cccctccccc gggatagccg cccgaaagga cgggtaatac cggatacccc ggggtgccgc 180

atggcgcccc ggctaaagcc ccgacgggag gggatggctc cgcggcccat caggtagacg 240

gcggggtgac ggcccaccgt gccgacaacg ggtagccggg ttgagagacc gaccggccag 300

attgggactg agacacggcc cagactccta cgggaggcag cagtggggaa tcttgcgcaa 360

tgggggaaac cctgacgcag cgacgccgcg tgcgggacgg aggccttcgg gtcgtaaacc 420

gctttcagca gggaagagtc aagactgtac ctgcagaaga agccccggct aactacgtgc 480

cagcagccgc ggtaatacgt agggggcgag cgttatccgg attcattggg cgtaaagcgc 540

gcgtaggcgg cccggcaggc cgggggtcga agcggggggc tcaacccccc gaagcccccg 600

gaacctccgc ggcttgggtc cggtagggag ggtggaacac cgggtgtagc ggtggaatgc 660

gcagatatcg ggtggaacac cgatggcgaa ggcggccctc tgggccaaga ccgacgctga 720

ggcgcgaaag ctgggggagc gaacaggatt agataccctg gtagtcccag ccgtaaacga 780

tggacgctag gtgtgggggg acgatccccc cgtgccgcag ccaacgcatt aagcgtcccg 840

cctggggagt acggccgcaa gntaaaactc aaaggaattg acgggggccc gcacaagcag 900

cggagcatgt ggcttaattc gaagcaacgc gaagaacctt accagggctt gacatatggg 960

tgaagcgggg gagacccgtg gccgagagga gcccatacag gtggtgcatg gctgtcgtca 1020

gctcgtgtcg tgagatgttg ggttaagtcc cgcaacgagc gcaacccccg ccgcgtgttg 1080

ccatcgggtg atgccgggaa cccacgcggg accgccgccg tcaaggggag gagggccggg 1140

acgacgtcaa gtcatcatgc cccttatgcc ctgggctgca cacgtgctac aatggccggt 1200

acagagggat gccaccccgc gaggggagcg gatcccggaa agccggcccc agttcggatt 1260

gggggctgca acccgccccc atgaagtcgg agttgctagt aaacgcggat cagcatgccg 1320

cggtgaatgc gttcccgggc cttgtacaca ccggccgtca caccacccga gtcgtctgca 1380

cccgaagtcg ccggcccaac cgagaggggg gaagcgccga aggtgtggaa ggttaagggg 1440

gttaagtcgt aacaaaggta acc 1463

<210> 12
<211> 1458
<212> DNA
<213> Collinsella

<220>
<221> misc_feature
<223> AB031061 S000382903

<400> 12
agagtttgat cctggctcag gatgaacgct ggcggcgcgc ctaacacatg caagtcgaac 60

ggcacccgcc ctcgggcgga agcgagtggc gaacggctga gtaacacgtg gagaacccgc 120

cccctcctcc gggatagcct cgggaaaccg tgggtaatac cggatgaccc cggcagccgc 180

atggccggcc ggcaaagccc agacgggagg ggatgctccg cggcccatca ggtagacggc 240

ggggtgacgg cccaccgtgc cgacgacggg tagccgggtt gagagaccga ccggccagat 300

tgggactgag acacggccca gactctacgg gaggcagcag tggggaatct tgcgcaatgg 360

ggggaaccct gacgcagcga cgccgcgtgc gggacgaagg ccctcgggtc gtaaaccctt 420

tcagcaggga tgagacaaga cggtacctgc agaagaagcc ccggctaact acgtgccagc 480

agccgcggta atacgtaggg ggcgaccgtt atccggattc attgggcgta aagcgcgcgt 540

aggcggcccg gcaggcaggg ggtcaaatgg cggggctcaa ccccgtcccg ccccctgaac 600

cgccgggctc gggtccggta ggggagggtg gaacacccgg tgtagcggtg gaatgcgcag 660

atatcgggtg gaacaccggt ggcgaagggg cctctgggcc gagaccgacg ctgaggcgcg 720

aaagctgggg gagcgaacag gattagatac cctggtagtc ccagccgtaa acgatggacg 780

ctaggtgtgg gaggggaacc cttccgtgcc gcagccaacg cattaacgtc ccgcctgggg 840

agtacggccg caaggctaaa actcaaagga attgacgggg gcccgcacaa gcagcggagc 900

atgtggctta attcgaagca acgcgaagaa ccttaccagg gcttgacatg cgcgtgaagc 960

cggggagacc cggtggccga gaggagcgcg cacaggtggt gcatggctgt cgtcagctcg 1020

tgtcgtgaga tgtttggtta agtccagcaa cgagcgcaac ccccgccgca tgttgccagc 1080

acctcgggtg ggcacccatg cgggaccgcc ggagtcaatc cggaggaggg cggggacgac 1140

gtcaagtcat catgcccctt atgccctggg ctgcacacgt gctacaatgg ccggtacagc 1200

gggatgcgat ggcgcgagcg gaggcggatc cctgaaagcc ggccccagtt cggattgggg 1260

gctgcaaccc gcccccatga agtcggagtt gctagtaatc gcggatcagc atgccgcggt 1320

gaatgcgttc ccgggccttg tacacaccgc ccgtcacacc acccgagtcg tctgcacccg 1380

aagtcgccgg cccaaccgag aggggggagg cgcctaaggt gttgagggtg aggggggtga 1440

agtcgtaaca aggtaacc 1458

<210> 13
<211> 1428
<212> DNA
<213> Subdoligranulum

<220>
<221> misc_feature
<223> AJ518869 S000416814

<220>
<221> misc_feature
<222> (1189)..(1189)
<223> n is a, c, g, or t

<400> 13
tgcaagtcga acggagttat ttcggttgaa gttttcggat ggatactggt ttaacttagt 60

ggcgaacggg tgagtaacgc gtgagtaacc tgccctggag tgggggacaa cagttggaaa 120

cgactgctaa taccgcataa gcccacgatc cggcatcgga ttgagggaaa aggatttatt 180

cgcttcagga tggactcgcg tccaattagc tagttggtga ggtaacggcc caccaaggcg 240

acgattggta gccggactga gaggttgaac ggccacattg ggactgagac acggcccaga 300

ctcctacggg aggcagcagt gggggatatt gcacaatggg ggaaaccctg atgcagcgac 360

gccgcgtgga ggaagaaggt tttcggattg taaactcctg tcgttaggga cgaatcttga 420

cggtacctaa caagaaagca ccggctaact acgtgccagc agccgcggta aaacgtaggg 480

tgcaagcgtt gtccggaatt actgggtgta aagggagcgc aggcggaccg gcaagttgga 540

agtgaaatct atgggctcaa cccataaatt gctttcaaaa ctgctggcct tgagtagtgc 600

agaggtaggt ggaattcccg gtgtagcggt ggaatgcgta gatatcggga ggaacaccag 660

tggcgaaggc gacctactgg gcaccaactg acgctgaggc tcgaaagcat gggtagcaaa 720

caggattaga taccctggta gtccatgccg taaacgatga ttactaggtg ttggaggatt 780

gaccccttca gtgccgcagt taacacaata agtaatccac ctggggagta cgaccgcaag 840

gttgaaactc aaaggaattg acgggggccc gcacaagcag tggagtatgt ggtttaattc 900

gaagcaacgc gaagaacctt accaggtctt gacatccgat gcatagtgca gagatgcatg 960

aagtccttcg ggacatcgag acaggtggtg catggttgtc gtcagctcgt gtcgtgagat 1020

gttgggttaa gtcccgcaac gagcgcaacc cttattgcca gttactacgc aagaggactc 1080

tggcgagact gccgttgaca aaacggagga aggtggggat gacgtcaaat catcatgccc 1140

tttatgacct gggctacaca cgtactacaa tggcgtttaa caaagagang caagaccgcg 1200

aggtggagca aaactcaaaa acaacgtctc agttcagatt gcaggctgca actcgcctgc 1260

atgaagtcgg aattgctagt aatcgcggat cagcatgccg cggtgaatac gttcccgggc 1320

cttgtacaca ccgcccgtca caccatgaga gccggggggg acccgaagtc ggtaagtaag 1380

tctaaccgca aggaggacgc cgccgaaggt aaaactggtg attgggtg 1428

<210> 14
<211> 1515
<212> DNA
<213> anaerostipes

<220>
<221> misc_feature
<223> FR749934 S002350797

<400> 14
tggctcagga tgaacgctgg cggcgtgctt aacacatgca agtcgaacga agctgcttaa 60

ctgatcttct tcggaattga cgttttgtag actgagtggc ggacgggtga gtaacgcgtg 120

ggcaacctgc cctgtacagg gggataacag tcagaaatga ctgctaatac cgcataagac 180

cacagcaccg catggtgcag gggtaaaaac tccggtggta caggatggac ccgcgtctga 240

ttagctggtt ggtgaggtaa cggctcacca aggcgacgat cagtagccgg cttgagagag 300

tgaacggcca cattgggact gagacacggc ccaaactcct acgggaggca gcagtgggga 360

atattgcaca atgggggaaa ccctgatgca gcgacgccgc gtgagtgaag aagtatctcg 420

gtatgtaaag ctctatcagc agggaagaaa atgacggtac ctgactaaga agccccggct 480

aactacgtgc cagcagccgc ggtaatacgt agggggcaag cgttatccgg aattactggg 540

tgtaaagggt gcgtaggtgg tatggcaagt cagaagtgaa aacccagggc ttaactctgg 600

gactgctttt gaaactgtca gactggagtg caggagaggt aagcggaatt cctagtgtag 660

cggtgaaatg cgtagatatt aggaggaaca tcagtggcga aggcggctta ctggactgaa 720

actgacactg aggcacgaaa gcgtggggag caaacaggat tagataccct ggtagtccac 780

gccgtaaacg atgaatacta ggtgtcgggg ccgtagaggc ttcggtgccg cagccaacgc 840

agtaagtatt ccacctgggg agtacgttcg caagaatgaa actcaaagga attgacgggg 900

acccgcacaa gcggtggagc atgtggttta attcgaagca acgcgaagaa ccttacctgg 960

tcttgacatc cttctgaccg gtccttaacc ggacctttcc ttcgggacag gagagacagg 1020

tggtgcatgg ttgtcgtcag ctcgtgtcgt gagatgttgg gttaagtccc gcaacgagcg 1080

caacccctat ctttagtagc cagcatttca ggtgggcact ctagagagac tgccagggat 1140

aacctggagg aaggtgggga cgacgtcaaa tcatcatgcc ccttatgacc agggctacac 1200

acgtgctaca atggcgtaaa cagagggaag cagcctcgtg agagtgagca aatcccaaaa 1260

ataacgtctc agttcggatt gtagtctgca actcgactac atgaagctgg aatcgctagt 1320

aatcgcgaat cagaatgtcg cggtgaatac gttcccgggt cttgtacaca ccgcccgtca 1380

caccatggga gtcagtaacg cccgaagtca gtgacccaac cgtaaggagg gagctgccga 1440

aggcgggacc gataactggg gtgaagtcgt aacaaggtag ccgtatcgga aggtgcggct 1500

ggatcacctc ctttc 1515

<210> 15
<211> 1381
<212> DNA
<213> anaerostipes

<220>
<221> misc_feature
<223> FJ947528 S001549427

<400> 15
ggctcaggat gaacgctggc ggcgtgctta acacatgcaa gtcgaacgaa gcttgatccc 60

ggatcccttc ggggtgacgg gtgatatgac tgagtggcgg acgggtgagt aacgcgtggg 120

caacctgcct catacagggg gataacagtt agaaatgact gctaagaccg cataagacca 180

cagcaccgca tggtggagtg gtaaaagctc cggtggtatg agatgggccc gcgtctgatt 240

agctggttgg tgaggtaacg gctctaccaa ggcaacgatc agtagccggc ttgagagagt 300

gaacggccac attgggactg agacacggcc caaactccta cgggaggcag cagtggggaa 360

tattgcacaa tgggggaaac cctgatgcag cgacgccgcg tgagtgaaga agtatttcgg 420

tatgtaaagc tctatcagca gggaagaaaa tggacggtac ctgactaaga agccccggct 480

aactacgtgc cagcagccgc ggtaatacgt agggggcaag cgttatccgg aattactggg 540

tgtaaagggt gcgtaggtgg cagggcaagt cagaagtgaa aacccggggc tcaactccgg 600

gactgctttt gaaactgtcc agctggagtg caggagaggt aagcggaatt cctagtgtag 660

cggtgaaatg cgtagatatt aggaggaaca tcagtggcga aggcggctta ctggactgaa 720

actgacactg aggcacgaaa gcgtggggag caaacaggat tagataccct ggtagtccac 780

gccgtaaacg atgaatacta ggtgtcgggg ccgtagaggc ttcggtgccg cagcaaacgc 840

attaagtatt ccacctgggg agtacgttcg caagaatgaa actcaaagga attgacgggg 900

acccgcacaa gcggtggagc atgtggttta attcgcaagc aacgcgaaga accttacctg 960

gtcttgacat ccccttgacc gctccttaat cggagttttc cttcgggaca agggtgacag 1020

gtggtgcatg gttgtcgtca gctcgtgtcg tgagatgttg ggttaagtcc cgcaacgagc 1080

gcaaccccta tctttagtag ccagcacggg aaaggtgggc actctagaga gactgccagg 1140

gagaacctgg aggaaggtgg gggacgacgt caaatcatca tgccccttat gaccagggct 1200

acacacgtgc tacaatggcg taaacaaagg gaagcgaccc tgtgaaggca agcaaatccc 1260

aaaaataacg tcacagttcg gattgtagtc tgcaactcga ctacatgaag ctggaatcgc 1320

tagtaatcgc gaatcagaat gtcgcggtga atacgttccc gggtcttgta cacaccgccc 1380

g 1381

<210> 16
<211> 1456
<212> DNA
<213> anaerostipes

<220>
<221> misc_feature
<223> AJ270487 S000088221

<400> 16
gcgcttaata catgtcaagt cgaacgaagc atttaggatt gaagttttcg gatggatttc 60

ctatatgact gagtggcgga cgggtgagta acgcgtgggg aacctgccct atacaggggg 120

ataacagctg gaaacggctg ctaataccgc ataagcgcac agaatcgcat gattcagtgt 180

gaaaagccct ggcagtatag gatggtcccg cgtctgatta gctggttggt gaggtaacgg 240

ctcaccaagg cgacgatcag tagccggctt gagagagtga acggccacat tgggactgag 300

acacggccca aactcctacg ggaggcagca gtggggaata ttgcacaatg ggggtaaacc 360

ctgatgcagc gacgccgcgt gagtgaagaa gtatttcggt atgtaaagct ctatcagcag 420

ggaagaaaac agacggtacc tgactaagaa gccccggcta actacgtgcc agcagccgcg 480

gtaatacgta gggggcaagc gttatccgga attactgggt gtaaagggtg cgtaggtggc 540

atggtaagtc agaagtgaaa gcccggggct taaccccggg actgctttgg aaactgtcat 600

gctggagtgc aggagaggta agcggaattc ctagtgtagc ggtgaaatgc gtagatatta 660

ggaggaacac cagtggcgaa ggcggcttac tggactgtca ctgacactga tgcacgaaag 720

cgtggggagc aaacaggatt agataccctg gtagtccacg ccgtaaacga tgaatactag 780

gtgtcggggc cgtagaggct tcggtgccgc agcaaacgca gtaagtattc cacctgggga 840

gtacgttcgc aagaatgaaa ctcaaaggaa ttgacgggga cccgcacaag cggtggagca 900

tgtggtttaa ttcgaagcaa cgcgaagaac cttacctggt cttgacatcc caatgaccga 960

accttaaccg gttttttctt tcgagacatt ggagacaggt ggtgcatggt tgtcgtcagc 1020

tcgtgtcgtg agatgttggg ttaagtcccg caacgagcgc aacccctatc tttagtagcc 1080

agcatttaag gtgggcactc tagagagact gccagggata acctggagga aggtggggac 1140

gacgtcaaat catcatgccc cttatggcca gggctacaca cgtgctacaa tggcgtaaac 1200

aaagggaagc gaagtcgtga ggcgaagcaa atcccagaaa taacgtctca gttcggattg 1260

tagtctgcaa ctcgactaca tgaagctgga atcgctagta atcgtgaatc agaatgtcac 1320

ggtgaatacg ttcccgggtc ttgtacacac cgcccgtcac accatgggag tcagtaacgc 1380

ccgaagtcag tgacccaacc gcaaggaggg agctgccgaa ggtgggaccg ataactgggg 1440

tgaagtcgta acaagg 1456

<210> 17
<211> 1616
<212> DNA
<213> anaerostipes

<220>
<221> misc_feature
<223> FR749932 S002350795

<400> 17
tttgatcctg gctcaggatg aacgctggcg gcgtgcttaa cacatgcaag tcgaacgaaa 60

caccttattt gattttcttc ggaactgaag atttggtgat tgagtggcgg acgggtgagt 120

aacgcgtggg taacctgccc tgtacagggg gataacagtc agaaatgact gctaataccg 180

cataagacca cagcaccgca tggtgcaggg gtaaaaactc cggtggtaca ggatggaccc 240

gcgtctgatt agctggttgg tgaggtaacg gctcaccaag gcgacgatca gtagccggct 300

tgagagagtg aacggccaca ttgggactga gacacggccc aaactcctac gggaggcagc 360

agtggggaat attgcacaat gggggaaacc ctgatgcagc gacgccgcgt gagtgaagaa 420

gtatctcggt atgtaaagct ctatcagcag ggaagaaaat gacggtacct gactaagaag 480

ccccggctaa ctacgtgcca gcagccgcgg taatacgtag ggggcaagcg ttatccggaa 540

ttactgggtg taaagggtgc gtaggtggta tggcaagtca gaagtgaaaa cccagggctt 600

aactctggga ctgcttttga aactgtcaga ctggagtgca ggagaggtaa gcggaattcc 660

tagtgtagcg gtgaaatgcg tagatattag gaggaacatc agtggcgaag gcggcttact 720

ggactgaaac tgacactgag gcacgaaagc gtggggagca aacaggatta gataccctgg 780

tagtccacgc cgtaaacgat gaatactagg tgtcggggcc gtaggggctt cggtgccgca 840

gccaacgcag taagtattcc acctggggag tacgttcgca agaatgaaac tcaaaggaat 900

tgacggggac ccgcacaagc ggtggagcat gtggtttaat tcgaagcaac gcgaagaacc 960

ttacctggtc ttgacatcct tctgaccggt ccttaaccgg acctttcctt cgggacagga 1020

gagacaggtg gtgcatggtt gtcgtcagct cgtgtcgtga gatgttgggt taagtcccgc 1080

aacgagcgca acccctatct ttagtagcca gcatataagg tgggcactct agagagactg 1140

ccagggataa cctggaggaa ggtggggacg acgtcaaatc atcatgcccc ttatgaccag 1200

ggctacacac gtgctacaat ggcgtaaaca gagggaagca gcctcgtgag agtgagcaaa 1260

tcccaaaaat aacgtctcag ttcggattgt agtctgcaac tcgactacat gaagctggaa 1320

tcgctagtaa tcgcgaatca gaatgtcgcg gtgaatacgt tcccgggtct tgtacacacc 1380

gcccgtcaca ccatgggagt cagtaacgcc cgaagtcagt gacccaaccg taaggaggga 1440

gctgccgaag gcgggaccga taactggggt gaagtcgtaa caaggtagcc gtatcggaag 1500

gtgcggctgg atcacctcct ttctaatccc gcggccatgg cggccgggag catgcgacgt 1560

cgggcccaat tcgccctata gtgagtcgta ttacaattca ctggccgtcg tttaaa 1616

<210> 18
<211> 1434
<212> DNA
<213> anaerostipes

<220>
<221> misc_feature
<223> JF412658 S002447232

<400> 18
gatgaacgct ggcggcgtgc ttaacacatg caagtcgaac gaaacacctt atttgatttt 60

cttcggaact gaagatttgg tgattgagtg gcggacgggt gagtaacgcg tgggtaacct 120

gccctgtaca gggggataac agtcagaaat gactgctaat accgcataag accacagcac 180

cgcatggtgc aggggtaaaa actccggtgg tacaggatgg acccgcgtct gattagctgg 240

ttggtgaggt aacggctcac caaggcgacg atcagtagcc ggcttgagag agtgaacggc 300

cacattggga ctgagacacg gcccaaactc ctacgggagg cagcagtggg gaatattgca 360

caatggggga aaccctgatg cagcgacgcc gcgtgagtga agaagtatct cggtatgtaa 420

agctctatca gcagggaaga aaatgacggt acctgactaa gaagccccgg ctaactacgt 480

gccagcagcc gcggtaatac gtagggggca agcgttatcc ggaattactg ggtgtaaagg 540

gtgcgtaggt ggtatggcaa gtcagaagtg aaaacccagg gcttaactct gggactgctt 600

ttgaaactgt cagactggag tgcaggagag gtaagcggaa ttcctagtgt agcggtgaaa 660

tgcgtagata ttaggaggaa catcagtggc gaaggcggct tactggactg aaactgacac 720

tgaggcacga aagcgtgggg agcaaacagg attagatacc ctggtagtcc acgccgtaaa 780

cgatgaatac taggtgtcgg ggccgtagag gcttcggtgc cgcagccaac gcagtaagta 840

ttccacctgg ggagtacgtt cgcaagaatg aaactcaaag gaattgacgg ggacccgcac 900

aagcggtgga gcatgtggtt taattcgaag caacgcgaag aaccttacct ggtcttgaca 960

tccttctgac cggtccttaa ccggaccttt ccttcgggac aggagagaca ggtggtgcat 1020

ggttgtcgtc agctcgtgtc gtgagatgtt gggttaagtc ccgcaacgag cgcaacccct 1080

atctttagta gccagcatat aaggtgggca ctctagagag actgccaggg ataacctgga 1140

ggaaggtggg gacgacgtca aatcatcatg ccccttatga ccagggctac acacgtgcta 1200

caatggcgta aacagaggga agcagcctcg tgagagtgag caaatcccaa aaataacgtc 1260

tcagttcgga ttgtagtctg caactcgact acatgaagct ggaatcgcta gtaatcgcga 1320

atcagaatgt cgcggtgaat acgttcccgg gtcttgtaca caccgcccgt cacaccatgg 1380

gagtcagtaa cgcccgaagt cagtgaccca accgtaagga gggagctgcc gaag 1434

<210> 19
<211> 1382
<212> DNA
<213> anaerostipes

<220>
<221> misc_feature
<223> JX273468 S003613922

<220>
<221> misc_feature
<222> (727)..(727)
<223> n is a, c, g, or t

<220>
<221> misc_feature
<222> (933)..(935)
<223> n is a, c, g, or t

<400> 19
tattttggat tgaagttttc ggatggatct ccttaatgac tgagtggcgg acgggtgagt 60

aacgcgtggg gaacctgccc tatacagggg gataacagct ggaaacggct gctaataccg 120

cataagcgca cagaatcgca tgattcagtg tgaaaagccc tggcagtata ggatggtccc 180

gcgtctgatt agctggttgg cggggtaacg gcccaccaag gcgacgatca gtagccggct 240

tgagagagtg gacggccaca ttgggactga gacacggccc aaactcctac gggaggcagc 300

agtggggaat attgcacaat gggggaaacc ctgatgcagc gacgccgcgt gagtgaagaa 360

gtatttcggt atgtaaagct ctatcagcag ggaagaaata agacggtacc tgactaagaa 420

gccccggcta actacgtgcc agcagccgcg gtaatacgta gggggcaagc gttatccgga 480

attactgggt gtaaagggtg cgtaggtggc atgataagtc agaagtgaaa gcccggggct 540

taaccccggg actgcttttg aaactgtaat gctagagtgc aggagaggta agcggaattc 600

ctagtgtagc ggtgaaatgc gtagatatta ggaggaacac cagtggcgaa ggcggcttac 660

tggactgtca ctgacactga ggcacgaaag cgtggggagc aaacaggatt agataccctg 720

gtagtcnacg ccgtaaacga tgaatactag gtgtcggggc cgtagaggct tcggtgccgc 780

agcaaacgca gtaagtattc cacctgggga gtacgttcgc aagaatgaaa ctcaaaggaa 840

ttgacgggga cccgcacaag cggtggagca tgtggtttaa ttcgaagcaa cgcgaagaac 900

cttacctggt cttgacatcc ttctgaccgg ttnnnaaccg aacctttcct tcgggacaga 960

agtgacaggt ggtgcatggt tgtcgtcagc tcgtgtcgtg agatgttggg ttaagtcccg 1020

caacgagcgc aacccctatc tttagtagcc agcatataag gtgggcactc tagagagact 1080

gccagggata acctggagga aggtggggac gacgtcaaat catcatgccc cttatggcca 1140

gggctacaca cgtgctacaa tggcgtaaac aaagggaagc gaccccgcga gggcaagcaa 1200

atcccagaaa taacgtctca gttcggattg tagtctgcaa ctcgactaca tgaagctgga 1260

atcgctagta atcgtgaatc agaatgtcac ggtgaatacg ttcccgggtc ttgtacacac 1320

cgcccgtcac accatgggag tcagtaacgc ccgaagtcag tgacccaacc gcaaggagga 1380

ga 1382

<210> 20
<211> 1433
<212> DNA
<213> akkermansia

<220>
<221> misc_feature
<223> AY271254 S000460661

<400> 20
aacgaacgct ggcggcgtgg ataagacatg caagtcgaac gagagaattg ctagcttgct 60

aataattctc tagtggcgca cgggtgagta acacgtgagt aacctgcccc cgagagcggg 120

atagccctgg gaaactggga ttaataccgc atagtatcga aagattaaag cagcaatgcg 180

cttggggatg ggctcgcggc ctattagtta gttggtgagg taacggctca ccaaggcgat 240

gacgggtagc cggtctgaga ggatgtccgg ccacactgga actgagacac ggtccagaca 300

cctacgggtg gcagcagtcg agaatcattc acaatggggg aaaccctgat ggtgcgacgc 360

cgcgtggggg aatgaaggtc ttcggattgt aaacccctgt catgtgggag caaattaaaa 420

agatagtacc acaagaggaa gagacggcta actctgtgcc agcagccgcg gtaatacaga 480

ggtctcaagc gttgttcgga atcactgggc gtaaagcgtg cgtaggctgt ttcgtaagtc 540

gtgtgtgaaa ggcgcgggct caacccgcgg acggcacatg atactgcgag actagagtaa 600

tggaggggga accggaattc tcggtgtagc agtgaaatgc gtagatatcg agaggaacac 660

tcgtggcgaa ggcgggttcc tggacattaa ctgacgctga ggcacgaagg ccaggggagc 720

gaaagggatt agatacccct gtagtcctgg cagtaaacgg tgcacgcttg gtgtgcgggg 780

aatcgacccc ctgcgtgccg gagtaacgcg ttaagcgtgc cgcctgggga gtacggtcgc 840

aagattaaaa ctcaaagaaa ttgacgggga cccgcacaag cggtggagta tgtggcttaa 900

ttcgatgcaa cgcgaagaac cttacctggg cttgacatgt aatgaacaac atgtgaaagc 960

atgcgactct tcggaggcgt tacacaggtg ctgcatggcc gtcgtcagct cgtgtcgtga 1020

gatgtttggt taagtccagc aacgagcgca acccctgttg ccagttacca gcacgtgaag 1080

gtggggactc tggcgagact gcccagatca actgggagga aggtggggac gacgtcaggt 1140

cagtatggcc cttatgccca gggctgcaca cgtactacaa tgcccagtac agagggggcc 1200

gaagccgcga ggcggaggaa atcctaaaaa ctgggcccag ttcggactgt aggctgcaac 1260

ccgcctacac gaagccggaa tcgctagtaa tggcgcatca gctacggcgc cgtgaatacg 1320

ttcccgggtc ttgtacacac cgcccgtcac atcatggaag ctggtcgcac ccgaagtatc 1380

tgaagccaac cgcaaggagg cagggtccta aggtgagact ggtaactggg atg 1433

<210> 21
<211> 1499
<212> DNA
<213> lactococcus

<220>
<221> misc_feature
<223> AB100803 S000439499

<400> 21
gacgaacgct ggcggcgtgc ctaatacatg caagttgagc gctgaaggtt ggtacttgta 60

ccgactggat gagcagcgaa cgggtgagta acgcgtgggg aatctgcctt tgagcggggg 120

acaacatttg gaaacgaatg ctaataccgc ataaaaactt taaacacaag ttttaagttt 180

gaaagatgca attgcatcac tcaaagatga tcccgcgttg tattagctag ttggtgaggt 240

aaaggctcac caaggcgatg atacatagcc gacctgagag ggtgatcggc cacattggga 300

ctgagacacg gcccaaactc ctacgggagg cagcagtagg gaatcttcgg caatggacga 360

aagtctgacc gagcaacgcc gcgtgagtga agaaggtttt cggatcgtaa aactctgttg 420

gtagagaaga acgttggtga gagtggaaag ctcatcaagt gacggtaact acccagaaag 480

ggacggctaa ctacgtgcca gcagccgcgg taatacgtag gtcccgagcg ttgtccggat 540

ttattgggcg taaagcgagc gcaggtggtt tattaagtct ggtgtaaaag gcagtggctc 600

aaccattgta tgcattggaa actggtagac ttgagtgcag gagaggagag tggaattcca 660

tgtgtagcgg tgaaatgcgt agatatatgg aggaacaccg gtggcgaaag cggctctctg 720

gcctgtaact gacactgagg ctcgaaagcg tggggagcaa acaggattag ataccctggt 780

agtccacgcc gtaaacgatg agtgctagat gtagggagct ataagttctc tgtatcgcag 840

ctaacgcaat aagcactccg cctggggagt acgaccgcaa ggttgaaact caaaggaatt 900

gacgggggcc cgcacaagcg gtggagcatg tggtttaatt cgaagcaacg cgaagaacct 960

taccaggtct tgacatactc gtgctattcc tagagatagg aagttccttc gggacacggg 1020

atacaggtgg tgcatggttg tcgtcagctc gtgtcgtgag atgttgggtt aagtcccgca 1080

acgagcgcaa cccctattgt tagttgccat cattaagttg ggcactctaa cgagactgcc 1140

ggtgataaac cggaggaagg tggggatgac gtcaaatcat catgcccctt atgacctggg 1200

ctacacacgt gctacaatgg atggtacaac gagtcgcgag acagtgatgt ttagctaatc 1260

tcttaaaacc attctcagtt cggattgtag gctgcaactc gcctacatga agtcggaatc 1320

gctagtaatc gcggatcagc acgccgcggt gaatacgttc ccgggccttg tacacaccgc 1380

ccgtcacacc acgggagttg ggagtacccg aagtaggttg cctaaccgca aggagggcgc 1440

ttcctaaggt aagaccgatg actggggtga agtcgtaaca aggtagccgt atcggaagg 1499

<210> 22
<211> 1499
<212> DNA
<213> lactococcus

<220>
<221> misc_feature
<223> AB100804 S000439500

<400> 22
gacgaacgct ggcggcgtgc ctaatacatg caagttgagc gctgaaggtt ggtacttgta 60

ccaactggat gagcagcgaa cgggtgagta acgcgtgggg aatctgcctt tgagtggggg 120

acaacatttg gaaacgaatg ctaataccgc ataaaaactt taaacacaag ttttaagttt 180

gaaagatgca attgcatcac tcaaagatga tcccgcgttg tattagctag ttggtgaggt 240

aaaggctcac caaggcgatg atacatagcc gacctgagag ggtgatcggc cacattggga 300

ctgagacacg gcccaaactc ctacgggagg cagcagtagg gaatcttcgg caatggacga 360

aagtctgacc gagcaacgcc gcgtgagtga agaaggtttt cggatcgtaa aactctgttg 420

gtagagaaga acgttggtga gagtggaaag ctcatcaagt gacggtaact acccagaaag 480

ggacggctaa ctacgtgcca gcagccgcgg taatacgtag gtcccgagcg ttgtccggat 540

ttattgggcg taaagcgagc gcaggtggtt tattaagtct ggtgtaaaag gcagtggctc 600

aaccattgta tgcattggaa actggtagac ttgagtgcag gagaggagag tggaattcca 660

tgtgtagcgg tgaaatgcgt agatatatgg aggaacaccg gtggcgaaag cggctctctg 720

gcctgtaact gacactgagg ctcgaaagcg tggggagcaa acaggattag ataccctggt 780

agtccacgcc gtaaacgatg agtgctagat gtagggagct ataagttctc tgtatcgcag 840

ctaacgcaat aagcactccg cctggggagt acgaccgcaa ggttgaaact caaaggaatt 900

gacgggggcc cgcacaagcg gtggagcatg tggtttaatt cgaagcaacg cgaagaacct 960

taccaggtct tgacatactc gtgctattcc tagagatagg aagttccttc gggacacggg 1020

atacaggtgg tgcatggttg tcgtcagctc gtgtcgtgag atgttgggtt aagtcccgca 1080

acgagcgcaa cccctattgt tagttgccat cattaagttg ggcactctaa cgagactgcc 1140

ggtgataaac cggaggaagg tggggatgac gtcaaatcat catgcccctt atgacctggg 1200

ctacacacgt gctacaatgg atggtacaac gagtcgcgag acagtgatgt ttagctaatc 1260

tcttaaaacc attctcagtt cggattgtag gctgcaactc gcctacatga agtcggaatc 1320

gctagtaatc gcggatcagc acgccgcggt gaatacgttc ccgggccttg tacacaccgc 1380

ccgtcacacc acgggagttg ggagtacccg aagtaggttg cctaaccgca aggagggcgc 1440

ttcctaaggt aagaccgatg actggggtga agtcgtaaca aggtagccgt atcggaagg 1499

<210> 23
<211> 1474
<212> DNA
<213> lactococcus

<220>
<221> misc_feature
<223> AB681296 S003263187

<400> 23
gacgaacgct ggcggcgtgc ctaatacatg caagtcgagc gatgattaaa gatagcttgc 60

tatttttatg aagagcggcg aacgggtgag taacgcgtgg gaaatctgcc gagtagcggg 120

ggacaacgtt tggaaacgaa cgctaatacc gcataacaat gagaatcgca tgattcttat 180

ttaaaagaag caattgcttc actacttgat gatcccgcgt tgtattagct agttggtagt 240

gtaaaggact accaaggcga tgatacatag ccgacctgag agggtgatcg gccacactgg 300

gactgagaca cggcccagac tcctacggga ggcagcagta gggaatcttc ggcaatgggg 360

gcaaccctga ccgagcaacg ccgcgtgagt gaagaaggtt ttcggatcgt aaaactctgt 420

tgttagagaa gaacgttaag tagagtggaa aattacttaa gtgacggtat ctaaccagaa 480

agggacggct aactacgtgc cagcagccgc ggtaatacgt aggtcccaag cgttgtccgg 540

atttattggg cgtaaagcga gcgcaggtgg tttcttaagt ctgatgtaaa aggcagtggc 600

tcaaccattg tgtgcattgg aaactgggag acttgagtgc aggagaggag agtggaattc 660

catgtgtagc ggtgaaatgc gtagatatat ggaggaacac cggaggcgaa agcggctctc 720

tggcctgtaa ctgacactga ggctcgaaag cgtggggagc aaacaggatt agataccctg 780

gtagtccacg ccgtaaacga tgagtgctag ctgtagggag ctataagttc tctgtagcgc 840

agctaacgca ttaagcactc cgcctgggga gtacgaccgc aaggttgaaa ctcaaaggaa 900

ttgacggggg cccgcacaag cggtggagca tgtggtttaa ttcgaagcaa cgcgaagaac 960

cttaccaggt cttgacatac tcgtgctatc cttagagata aggagttcct tcgggacacg 1020

ggatacaggt ggtgcatggt tgtcgtcagc tcgtgtcgtg agatgttggg ttaagtcccg 1080

caacgagcgc aacccttatt actagttgcc atcattaagt tgggcactct agtgagactg 1140

ccggtgataa accggaggaa ggtggggatg acgtcaaatc atcatgcccc ttatgacctg 1200

ggctacacac gtgctacaat ggatggtaca acgagtcgcc aacccgcgag ggtgcgctaa 1260

tctcttaaaa ccattctcag ttcggattgc aggctgcaac tcgcctgcat gaagtcggaa 1320

tcgctagtaa tcgcggatca gcacgccgcg gtgaatacgt tcccgggcct tgtacacacc 1380

gcccgtcaca ccacggaagt tgggagtacc caaagtaggt tgcctaaccg caaggagggc 1440

gcttcctaag gtaagaccga tgactggggt gaag 1474

<210> 24
<211> 1433
<212> DNA
<213> lactococcus

<220>
<221> misc_feature
<223> EF694029 S000893640

<400> 24
cctaatacat gcaagttgaa cgctgaagac tggtacttgt actagttgga tgagtagcga 60

acgggtgagt aacgcgtggg taacctacct cttagcgggg gacaactatt ggaaacgata 120

gctaataccg cataacaata tttaacacat gttaagtatt tgaaagcacc aattggtgca 180

ctaagagatg gacccgcgtt gtattagcta gttggtagtg taatggacta ccaaggcgat 240

gatacatagc cgacctgaga gggtgatcgg ccacattggg actgagacac ggcccaaact 300

cctacgggag gcagcagtag ggaatcttcg gcaatggacg aaagtctgac cgagcaacgc 360

cgcgtgagtg aagaaggttt tcggatcgta aaactctgtt gttagagaag aacgtcagtg 420

agagtggaaa gttcactgag tgacggtatc taaccagaaa gggacggcta actacgtgcc 480

agcagccgcg gtaatacgta ggtcccgagc gttgtccgga tttattgggc gtaaagcgag 540

cgcaggtggt ttaataagtc tgatgtaaaa ggcagtggct caaccattgt gtgcattgga 600

aactgttaga cttgagtgca gtagaggaga gtggaattcc atgtgtagcg gtgaaatgcg 660

tagatatatg gaggaacacc ggtggcgaaa gcggctctct ggactgtaac tgacactgag 720

gctcgaaagc gtgggtagca aacaggatta gataccctgg tagtccacgc cgtaaacgat 780

gagtgctagt tgtttggggc tatccagccc taagtgacgc agcaaacgca ttaagcactc 840

cgcctgggga gtacgaccgc aaggttgaaa ctcaaaggaa ttgacggggg cccgcacaag 900

cggtggagca tgtggtttaa ttcgaagcaa cgcgaagaac cttaccaggt cttgacatac 960

cagtgctatt cttagagata agaagttact tcggtacact ggatacaggt ggtgcatggt 1020

tgtcgtcagc tcgtgtcgtg agatgttggg ttaagtcccg caacgagcgc aacccctatt 1080

gttagttgcc atcattaagt tgggcactct agcgagactg ccggtaataa accggaggaa 1140

ggtggggatg acgtcaaatc atcatgcccc ttatgacctg ggctacacac gtgctacaat 1200

ggttggtaca acgagtcgca agctggtgac agcaagctaa tctcttaaag ccaatctcag 1260

ttcggattgt aggctgcaac tcgcctacat gaagtcggaa tcgctagtaa tcgcggatca 1320

gcacgccgcg gtgaatacgt tcccgggcct tgtacacacc gcccgtcaca ccacgagagt 1380

ttgtaatacc caaagccggt gagctaacct ttatggaggc agccgtctaa ggt 1433

<210> 25
<211> 1436
<212> DNA
<213> lactococcus

<220>
<221> misc_feature
<223> DQ343754 S000640753

<400> 25
ggtacttgta tcagaatgaa gagtagcgaa cgggtgagta acgcgtgggt aacctacctc 60

ttagcggggg acaactattg gaaacgatag ctaataccgc ataacaattc ttaacacatg 120

ttaagaattt gaaagtacca attggtacac taggagatgg acccgcgttg tattagctag 180

ttggtagtgt aatggactac caaggcgatg atacatagcc gacctgagag ggtgatcggc 240

cacattggga ctgagacacg gcccaaactc ctacgggagg cagcagtagg gaatcttcgg 300

caatggacga aagtctgacc gagcaacgcc gcgtgagtga agaaggtttt cggatcgtaa 360

aactctgttg ttagagaaga acgttgtgta gagtggaaaa ttacacaagt gacggtatct 420

aaccagaaag ggacggctaa ctacgtgcca gcagccgcgg taatacgtag gtcccgagcg 480

ttgtccggat ttattgggcg taaagcgagc gcaggtggtt taataagtct gatgtaaaag 540

gcagtggctc aaccattgtg tgcattggaa actgttagac ttgagtgcag tagaggagag 600

tggaattcca tgtgtagcgg tgaaatgcgt agatatatgg aggaacaccg gtggcgaaag 660

cggctctctg gactgtaact gacactgagg ctcgaaagcg tgggtagcaa acaggattag 720

ataccctggt agtccacgcc gtaaacgatg agtgctagtt gtttggggct atccagccct 780

aagtgacgca gcaaacgcat taagcactcc gcctggggag tacgaccgca aggttgaaac 840

tcaaaggaat tgacgggggc ccgcacaagc ggtggagcat gtggtttaat tcgaagcaac 900

gcgaagaacc ttaccaggtc ttgacatacc agtgctattc ttagagataa gaagttactt 960

cggtacactg gatacaggtg gtgcatggtt gtcgtcagct cgtgtcgtga gatgttgggt 1020

taagtcccgc aacgagcgca acccctattg ttagttgcca tcattaagtt gggcactcta 1080

gcgagactgc cggtaataaa ccggaggaag gtggggatga cgtcaaatca tcatgcccct 1140

tatgacctgg gctacacacg tgctacaatg gttggtacaa cgagtcgcaa gctagtgata 1200

gcaagctaat ctcttaaagc caatctcagt tcggattgta ggctgcaact cgcctacatg 1260

aagtcggaat cgctagtaat cgcggatcag cacgccgcgg tgaatacgtt cccgggcctt 1320

gtacacaccg cccgtcacac cacgagagtt tgtaataccc aaagccggtg agctaacctt 1380

ttaggaggca gccgtctaag gtaggataga tgattggggt gaagcgtaac aagtag 1436

<210>  26
<211>  1541
<212>  DNA
<213>  lactococcus

<220>
<221>  misc_feature
<223>  EU770697  S001153507

<220>
<221>  misc_feature
<222>  (1523)..(1523)
<223>  n is a, c, g, or t

<400>  26
tagagtttga tcctggctca ggacgaacgc tggcggcgtg cctaatacat gcaagttgag      60

cgatgaagat tggtgcttgc accaatttga agagcagcga acgggtgagt aacgcgtggg      120

gaatctgcct ttgagcgggg gacaacattt ggaaacgaat gctaataccg cataacaact      180

ttaaacacaa gttttaagtt tgaaagatgc aattgcatca ctcaaagatg atcccgcgtt      240

gtattagcta gttggtgagg taaaggctca ccaaggcgat gatacatagc cgacctgaga      300

gggtgatcgg ccacattggg actgagacac ggcccaaact cctacgggag gcagcagtag      360

ggaatcttcg gcaatggacg aaagtctgac cgagcaacgc cgcgtgagtg aagaaggttt      420

tcggatcgta aaactctgtt ggtagagaag aacgttggtg agagtggaaa gctcatcaag      480

tgacggtaac tacccagaaa gggacggcta actacgtgcc agcagccgcg gtaatacgta      540

ggtcccgagc gttgtccgga tttattgggc gtaaagcgag cgcaggtggt ttattaagtc      600

tggtgtaaaa ggcagtggct caaccattgt atgcattgga aactggtaga cttgagtgca      660

ggagaggaga gtggaattcc atgtgtagcg gtgaaatgcg tagatatatg gaggaacacc      720

ggtggcgaaa gcggctctct ggcctgtaac tgacactgag gctcgaaagc gtggggagca      780

aacaggatta gataccctgg tagtccacgc cgtaaacgat gagtgctaga tgtagggagc      840

tataagttct ctgtatcgca gctaacgcaa taagcactcc gcctggggag tacgaccgca      900

aggttgaaac tcaaaggaat tgacgggggc ccgcacaagc ggtggagcat gtggtttaat      960

tcgaagcaac gcgaagaacc ttaccaggtc ttgacatact cgtgctattc ctagagatag      1020

gaagttcctt cgggacacgg gatacaggtg gtgcatggtt gtcgtcagct cgtgtcgtga      1080

gatgttgggt taagtcccgc aacgagcgca acccctattg ttagttgcca tcattaagtt      1140

gggcactcta acgagactgc cggtgataaa ccggaggaag gtggggatga cgtcaaatca      1200

tcatgcccct tatgacctgg gctacacacg tgctacaatg gatggtacaa cgagtcgcga      1260

gacagtgatg tttagctaat ctcttaaaac cattctcagt tcggattgta ggctgcaact      1320

cgcctacatg aagtcggaat cgctagtaat cgcggatcag cacgccgcgg tgaatacgtt      1380

cccgggcctt gtacacaccg cccgtcacac cacgggagtt gggagtaccc gaagtaggtt 1440

gcctaaccgc aaggagggcg cttcctaagg taagaccgat gactggggtg aagtcgtaac 1500

aaggtagccg tatcggaagg tgnggatgga tcacctcctt a 1541

<210> 27
<211> 1499
<212> DNA
<213> lactococcus

<220>
<221> misc_feature
<223> AB100802 S000439498

<400> 27
gacgaacgct ggcggcgtgc ctaatacatg caagttgagc gatgaagatt ggtgcttgca 60

ccaatttgaa gagcagcgaa cgggtgagta acgcgtgggg aatctgcctt tgagcggggg 120

acaacatttg gaaacgaatg ctaataccgc ataacaactt taaacataag ttttaagttt 180

gaaagatgca attgcatcac tcaaagatga tcccgcgttg tattagctag ttggtgaggt 240

aaaggctcac caaggcgatg atacatagcc gacctgagag ggtgatcggc cacattggga 300

ctgagacacg gcccaaactc ctacgggagg cagcagtagg gaatcttcgg caatggacga 360

aagtctgacc gagcaacgcc gcgtgagtga agaaggtttt cggatcgtaa aactctgttg 420

gtagagaaga acgttggtga gagtggaaag ctcatcaagt gacggtaact acccagaaag 480

ggacggctaa ctacgtgcca gcagccgcgg taatacgtag gtcccgagcg ttgtccggat 540

ttattgggcg taaagcgagc gcaggtggtt tattaagtct ggtgtaaaag gcagtggctc 600

aaccattgta tgcattggaa actggtagac ttgagtgcag gagaggagag tggaattcca 660

tgtgtagcgg tgaaatgcgt agatatatgg aggaacaccg gtggcgaaag cggctctctg 720

gcctgtaact gacactgagg ctcgaaagcg tggggagcaa acaggattag ataccctggt 780

agtccacgcc gtaaacgatg agtgctagat gtagggagct ataagttctc tgtatcgcag 840

ctaacgcaat aagcactccg cctggggagt acgaccgcaa ggttgaaact caaaggaatt 900

gacgggggcc cgcacaagcg gtggagcatg tggtttaatt cgaagcaacg cgaagaacct 960

taccaggtct tgacatactc gtgctattcc tagagatagg aagttccttc gggacacggg 1020

atacaggtgg tgcatggttg tcgtcagctc gtgtcgtgag atgttgggtt aagtcccgca 1080

acgagcgcaa cccctattgt tagttgccat cattaagttg ggcactctaa cgagactgcc 1140

ggtgataaac cggaggaagg tggggatgac gtcaaatcat catgcccctt atgacctggg 1200

ctacacacgt gctacaatgg atggtacaac gagtcgcgag acagtgatgt ttagctaatc 1260

tcttaaaacc attctcagtt cggattgtag gctgcaactc gcctacatga agtcggaatc 1320

gctagtaatc gcggatcagc acgccgcggt gaatacgttc ccgggccttg tacacaccgc 1380

ccgtcacacc acgggagttg ggagtacccg aagtaggttg cctaaccgca aggagggcgc 1440

ttcctaaggt aagaccgatg actggggtga agtcgtaaca aggtagccgt atcggaagg 1499

<210> 28
<211> 1506
<212> DNA
<213> lactococcus

<220>
<221> misc_feature
<223> AB485959 S002949140

<400> 28
agagtttgat cctggctcag gacgaacgct ggcggcgtgc ctaatacatg caagtcgagc 60

gatgataatt tagcttgcta aatttgacga gcggcgaacg ggtgagtaac gcgtggggaa 120

tctgccgagg agcgggggac aacaaaagga aacgaatgct aataccgcat aaaagcttga 180

aagacatctt tcgagtttga aaggaacaat tgtttcactc ctcgatgatc ccgcgttgta 240

ttagctagtt ggtagggtaa cggcctacca aggcgatgat acatagccga cctgagaggg 300

tgatcggcca cattgggact gagacacggc ccaaactcct acgggaggca gcagtaggga 360

atcttcggca atggacgaaa gtctgaccga gcaacgccgc gtgagtgaag aaggttttcg 420

gatcgtaaag ctctgttgtt gttaaagaac gtctgtgaga gtggaaagtt cacagagtga 480

cggtaaacaa ccagaaaggg acggctaact acgtgccagc agccgcggta atacgtaggt 540

cccgagcgtt gtccggattt attgggcgta aagcgagcgc aggtggtttc ttaagtctgg 600

tgtaaaaggc tactgctcaa cagtagtgtg cattggaaac tgggagactt gagtgcagga 660

gaggagagtg gaattccatg tgtagcggtg aaatgcgtag atatatggag gaacaccgga 720

ggcgaaagcg gctctctggc ctgtaactga cactgaggct cgaaagcgtg gggagcaaac 780

aggattagat accctggtag tccacgccgt aaacgatgag tgctagatgt gggaagctat 840

aagttttccg tatcgcagct aacgcaataa gcactccgcc tggggagtac gaccgcaagg 900

ttgaaactca aaggaattga cgggggcccg cacaagcggt ggagcatgtg gtttaattcg 960

aagcaacgcg aagaacctta ccaggtcttg acatactgat gctatcctta gagataagga 1020

gttccttcgg gacatcggat acaggtggtg catggttgtc gtcagctcgt gtcgtgagat 1080

gttgggttaa gtcccgcaac gagcgcaacc cctattacta gttgccatca ttaagttggg 1140

cactctagtg agactgccag tgataaaccg gaggaaggtg gggatgacgt caaatcatca 1200

tgccccttat gacctgggct acacacgtgc tacaatggat ggtacaacga gtcgcgagac 1260

agtgatgttt agctaatctc ttaaaaccat tctcagttcg gattgtaggc tgcaactcgc 1320

ctacatgaag tcggaatcgc tagtaatcgc ggatcagcac gccgcggtga atacgttccc 1380

gggccttgta cacaccgccc gtcacaccat gggagttggg agtacccgaa gtaggttgcc 1440

taaccgtaag gagggcgctt cctaaggtaa gaccgatgac tggggtgaag tcgtaacaag 1500

gtaacc 1506

<210> 29
<211> 1510
<212> DNA
<213> lactococcus

<220>
<221> misc_feature
<223> AB598994 S003316271

<400> 29
agagtttgat cctggctcag gacgaacgct ggcggcgtgc ctaatacatg caagtcgagc 60

gatgattaaa gatagcttgc tatttttatg aagagcggcg aacgggtgag taacgcgtgg 120

gaaatctgcc gagtagcggg ggacaacgtt tggaaacgaa cgctaatacc gcataacaat 180

gagaatcgca tgattcttat ttaaaagaag caattgcttc actacttgat gatcccgcgt 240

tgtattagct agttggtagt gtaaaggact accaaggcga tgatacatag ccgacctgag 300

agggtgatcg gccacactgg gactgagaca cggcccagac tcctacggga ggcagcagta 360

gggaatcttc ggcaatgggg gcaaccctga ccgagcaacg ccgcgtgagt gaagaaggtt 420

ttcggatcgt aaaactctgt tgttagagaa gaacgttaag tagagtggaa aattacttaa 480

gtgacggtat ctaaccagaa agggacggct aactacgtgc cagcagccgc ggtaatacgt 540

aggtcccaag cgttgtccgg atttattggg cgtaaagcga gcgcaggtgg tttcttaagt 600

ctgatgtaaa aggcagtggc tcaaccattg tgtgcattgg aaactgggag acttgagtgc 660

aggagaggag agtggaattc catgtgtagc ggtgaaatgc gtagatatat ggaggaacac 720

cggaggcgaa agcggctctc tggcctgtaa ctgacactga ggctcgaaag cgtggggagc 780

aaacaggatt agataccctg gtagtccacg ccgtaaacga tgagtgctag ctgtagggag 840

ctataagttc tctgtagcgc agctaacgca ttaagcactc cgcctgggga gtacgaccgc 900

aaggttgaaa ctcaaaggaa ttgacggggg cccgcacaag cggtggagca tgtggtttaa 960

ttcgaagcaa cgcgaagaac cttaccaggt cttgacatac tcgtgctatc cttagagata 1020

aggagttcct tcgggacacg ggatacaggt ggtgcatggt tgtcgtcagc tcgtgtcgtg 1080

agatgttggg ttaagtcccg caacgagcgc aacccttatt actagttgcc atcattaagt 1140

tgggcactct agtgagactg ccggtgataa accggaggaa ggtggggatg acgtcaaatc 1200

atcatgcccc ttatgacctg ggctacacac gtgctacaat ggatggtaca acgagtcgcc 1260

aacccgcgag ggtgcgctaa tctcttaaaa ccattctcag ttcggattgc aggctgcaac 1320

tcgcctgcat gaagtcggaa tcgctagtaa tcgcggatca gcacgccgcg gtgaatacgt 1380

tcccgggcct tgtacacacc gcccgtcaca ccacggaagt tgggagtacc caaagtaggt 1440

tgcctaaccg caaggagggc gcttcctaag gtaagaccga tgactggggt gaagtcgtac 1500

aaaggtaacc 1510

<210> 30
<211> 1463
<212> DNA
<213> lactococcus

<220>
<221> misc_feature
<223> AB699722 S003265167

<400> 30
cagggtttga gtatttgctc aggacgaacg ctggcggcgt gcctaataca tgcaagttga 60

gcgctgaaga ttggtacttg taccgattgg atgagcagcg aacgggtgag taacgcgtgg 120

ggaatctgcc tttgagcggg ggacaacatt tggaaacgaa tgctaatacc gcataatagc 180

tttaaacata agttttaagt ttgaaagatg caattgcatc actcaaagat gatcccgcgt 240

tgtattagct agttggtgag gtaaaggctc accaaggcga tgatacatag ccgacctgag 300

agggtgatcg gccacattgg gactgagaca cggcccaaac tcctacggga ggcagcagta 360

gggaatcttc ggcaatggac gaaagtctga ccgagcaacg ccgcgtgagt gaagaaggtt 420

ttcggatcgt aaaactctgt tgttagagaa gaacgacgag gagagtggaa agctccttgt 480

gtgacggtat ctaaccagaa agggacggct aactacgtgc cagcagccgc ggtaatacgt 540

aggtcccgag cgttgtccgg atttattggg cgtaaagcga gcgcaggtgg tttattaagt 600

ctggtgtaaa aggcagtggc tcaaccattg tatgcattgg aaactggtag acttgagtgc 660

aggagaggag agtggaattc catgtgtagc ggtgaaatgc gtagatatat ggaggaacac 720

cggtggcgaa agcggctctc tggcctgtaa ctgacactga ggctcgaaag cgtggggagc 780

aaacaggatt agataccctg gtagtccacg ccgtaaacga tgagtgctag atgtagggag 840

ctataagttc tctgtatcgc agctaacgca ataagcactc cgcctgggga gtacgaccgc 900

aaggttgaaa ctcaaaggaa ttgacggggg cccgcacaag cggtggagca tgtggtttaa 960

ttcgaagcaa cgcgaagaac cttaccaggt cttgacatac tcgtgctatt cctagagata 1020

ggaagttcct tcgggacacg ggatacaggt ggtgcatggt tgtcgtcagc tcgtgtcgtg 1080

agatgttggg ttaagtcccg caacgagcgc aacccctatt gttagttgcc atcattaagt 1140

tgggcactct aacgagactg ccggtgataa accggaggaa ggtggggatg acgtcaaatc 1200

atcatgcccc ttatgacctg ggctacacac gtgctacaat ggatggtaca acgagtcgcg 1260

agacagtgat gtttagctaa tctcttaaaa ccattctcag ttcggattgt aggctgcaac 1320

tcgcctacat gaagtcggaa tcgctagtaa tcgcggatca gcacgccgcg gtgaatacgt 1380

tcccgggcct tgtacacacc gcccgtcaca ccacgggagt tgggagtacc cgaagttggt 1440

tgcctaaccg caaggagggc gct 1463

<210> 31
<211> 1515
<212> DNA
<213> lactococcus

<220>
<221> misc_feature
<223> AB775178 S003659973

<400> 31
tttgagtttt gatctggctc aggacgaacg ctggcggcgt gcctaataca tgcaagtcga 60

gcgatgattg aagatagctt gctattttca tgaagagcgg cgaacgggtg agtaacgcgt 120

gggaaatctg ccgagtagcg ggggacaacg tttggaaacg aacgctaata ccgcataaca 180

atgagaatcg catgattctt atttgaaaga agcaattgct tcactacttg atgatcccgc 240

gttgtattag ctagttggta gtgtaaagga ctaccaaggc gacgatacat agccgacctg 300

agagggtgat cggccacact gggactgaga cacggcccag actcctacgg gaggcagcag 360

tagggaatct tcggcaatgg gggcaaccct gaccgagcaa cgccgcgtga gtgaagaagg 420

ttttcggatc gtaaaactct gttgttagag aagaacgtta agtagagtgg aaaattactt 480

aagtgacggt atctaaccag aaagggacgg ctaactacgt gccagcagcc gcggtaatac 540

gtaggtccca agcgttgtcc ggatttattg ggcgtaaagc gagcgcaggt ggtttcttaa 600

gtctgatgta aaaggcagtg gctcaaccat tgtgtgcatt ggaaactggg agacttgagt 660

gcaggagagg agagtggaat tccatgtgta gcggtgaaat gcgtagatat atggaggaac 720

accggaggcg aaagcggctc tctggcctgt aactgacact gaggctcgaa agcgtgggga 780

gcaaacagga ttagataccc tggtagtcca cgccgtaaac gatgagtgct agctgtaggg 840

agctataagt tctctgtagc gcagctaacg cattaagcac tccgcctggg gagtacgacc 900

gcaaggttga aactcaaagg aattgacggg ggcccgcaca agcggtggag catgtggttt 960

aattggaagc aacgcgaaga accttaccag gtcttgacat cccgatgcta tccttagaga 1020

taaggagtta cttcggtaca tcggtgacag gtggtgcatg gttgtcgtca gctcgtgtcg 1080

tgagatgttg ggttaagtcc cgcaacgagc gcaaccctta ttactagttg ccatcattaa 1140

gttgggcact ctagtgagac tgccggtgat aaaccggagg aaggtgggga tgacgtcaaa 1200

tcatcatgcc ccttatgacc tgggctacac acgtgctaca atggatggta caacgagtcg 1260

ccaacccgcg agggtgcgct aatctcttaa aaccattctc agttcggatt gcaggctgca 1320

actcgcctgc atgaagtcgg aatcgctagt aatcgcggat cagcacgccg cggtgaatac 1380

gttcccgggc cttgtacaca ccgcccgtca caccacggaa gttgggagta cccaaagtag 1440

gttgcctaac cgcaaggagg gcgcttccta aggtaagacc gatgactggg gtgaagtcga 1500

aaaaggggta aacaa 1515

<210> 32
<211> 1440
<212> DNA
<213> lactococcus

<220>
<221> misc_feature
<223> EF694030 S000893641

<400> 32
agttgaacgc ttgattttca ccgaagcttg cttcaccgaa aatcaagagt agcgaacggg 60

tgagtaacgc gtgggtaacc tacctatcag cgggggataa ctattggaaa cgatagctaa 120

taccgcataa caatgttgga tgcatattcg acatttgaaa gtaccaaatg gtacactgag 180

agatggaccc gcgttgtatt agctagttgg tagtgtaatg gactaccaag gcgatgatac 240

atagccgacc tgagagggtg atcggccaca ttgggactga gacacggccc aaactcctac 300

gggaggcagc agtagggaat cttcggcaat ggacgaaagt ctgaccgagc aacgccgcgt 360

gagtgaagaa ggttttcgga tcgtaaaact ctgttgttag agaagaacgt tgcatagagt 420

ggaaaattat gcaagtgacg gtatctaacc agaaagggac ggctaactac gtgccagcag 480

ccgcggtaat acgtaggtcc cgagcgttgt ccggatttat tgggcgtaaa gcgagcgcag 540

gtggtttaat aagtctgatg taaaaggcag tggctcaacc attgtgtgca ttggaaactg 600

ttagacttga gtgcagtaga ggagagtgga attccatgtg tagcggtgaa atgcgtagat 660

atatggagga acaccggtgg cgaaagcggc tctctggact gtcactgaca ctgaggctcg 720

aaagcgtggg tagcaaacag gattagatac cctggtagtc cacgccgtaa acgatgagtg 780

ctagttgttt ggggctatcc agccctaagt gacgcagcaa acgcattaag cactccgcct 840

ggggagtacg accgcaaggt tgaaactcaa aggaattgac gggggcccgc acaagcggtg 900

gagcatgtgg tttaattcga agcaacgcga agaaccttac caggtcttga catactcgtg 960

ctattcctag agataggaag ttccttcggg acacgggata caggtggtgc atggttgtcg 1020

tcagctcgtg tcgtgagatg ttgggttaag tcccgcaacg agcgcaaccc ctattgttag 1080

ttgccatcat taagttgggc actctagcga gactgccggt aataaaccgg aggaaggtgg 1140

ggatgacgtc aaatcatcat gccccttatg acctgggcta cacacgtgct acaatggttg 1200

gtacaacgag tcgcaaggca gtgatgtcaa gctaatctct taaagccaat ctcagttcgg 1260

attgtaggct gcaactcgcc tacatgaagt cggaatcgct agtaatcgcg gatcagcacg 1320

ccgcggtgaa tacgttcccg ggccttgtac acaccgcccg tcacaccacg agagtttgta 1380

atacccaaag ccggtgagct aaccttttag gaggcagccg tctaaggtag gatagatgat 1440

<210> 33
<211> 1463
<212> DNA
<213> lactococcus

<220>
<221> misc_feature
<223> EF694028 S000893639

<400> 33
agctatcatg caagttgaac gcaacaaaga caccagagct tgctctaccg tctttgttga 60

gtagcgaacg ggtgagtaac gcgtgggtaa cctgccttat agcgggggat aactattgga 120

aacgatagct aataccgcat aacaatagag attgcataat ctttatttga aagtgccaac 180

tggtacacta tgagatggac ccgcgttgta ttagctagtt ggtagtgtaa tggactacca 240

aggcgatgat acatagccga cctgagaggg tgatcggcca cattgggact gagacacggc 300

ccaaactcct acgggaggca gcagtaggga atcttcggca atggacgaaa gtctgaccga 360

gcaacgccgc gtgagtgaag aaggttttcg gatcgtaaaa ctctgttgtt agagaagaac 420

gttgattaga gtggaaaatt aatcaagtga cggtatctaa ccagaaaggg acggctaact 480

acgtgccagc agccgcggta atacgtaggt cccgagcgtt gtccggattt attgggcgta 540

aagcgagcgc aggtggttta ataagtctga tgtaaaaggc agtggctcaa ccattgtgtg 600

cattggaaac tgttagactt gagtgcagta gaggagagtg gaattccatg tgtagcggtg 660

aaatgcgtag atatatggag gaacaccggt ggcgaaagcg gctctctgga ctgtcactga 720

cactgaggct cgaaagcgtg ggtagcaaac aggattagat accctggtag tccacgccgt 780

aaacgatgag tgctagttgt ttggggctat ccagccctaa gtgacgcagc aaacgcatta 840

agcactccgc ctggggagta cgaccgcaag gttgaaactc aaaggaattg acgggggccc 900

gcacaagcgg tggagcatgt ggtttaattc gaagcaacgc gaagaacctt accaggtctt 960

gacatactcg tgctattcct agagatagga agttccttcg ggacacggga tacaggtggt 1020

gcatggttgt cgtcagctcg tgtcgtgaga tgttgggtta agtcccgcaa cgagcgcaac 1080

ccttattgtt agttgccatc attaagttgg gcactctagc gagactgccg gtaataaacc 1140

ggaggaaggt ggggatgacg tcaaatcatc atgcccctta tgacctgggc tacacacgtg 1200

ctacaatggt tggtacaacg agtcgcaaga cagtgatgtc aagctaatct cttaaagcca 1260

atctcagttc ggattgtagg ctgcaactcg cctacatgaa gtcggaatcg ctagtaatcg 1320

cggatcagca cgccgcggtg aatacgttcc cgggccttgt acacaccgcc cgtcacacca 1380

cgagagtttg taatacccaa agccggtgag ctaacctttt aggaggcagc cgtctaaggt 1440

aggatagatg attgggggaa gtc 1463